(19) Europäisches Patentamt
European Patent Office
Office européen des brevets

(11) **EP 4 282 880 A1**

(12) **EUROPEAN PATENT APPLICATION**
published in accordance with Art. 153(4) EPC

(43) Date of publication:
**29.11.2023 Bulletin 2023/48**

(21) Application number: **22736536.8**

(22) Date of filing: **05.01.2022**

(51) International Patent Classification (IPC):
**C07K 16/10** [(2006.01)]  **C07K 7/04** [(2006.01)]
**C07K 14/165** [(2006.01)]  **C12N 15/13** [(2006.01)]
**C12N 15/85** [(2006.01)]  **A61K 39/42** [(2006.01)]
**A61P 31/14** [(2006.01)]

(52) Cooperative Patent Classification (CPC):
**A61K 39/215; A61K 39/42; A61K 45/00;
A61K 47/68; A61P 31/14; C07K 7/04;
C07K 14/165; C07K 16/00; C07K 16/10;
C12N 15/85; G01N 33/569**

(86) International application number:
**PCT/CN2022/070309**

(87) International publication number:
**WO 2022/148374 (14.07.2022 Gazette 2022/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.01.2021 CN 202110008031**

(71) Applicant: **Center for Excellence in Molecular Cell
Science,
Chinese Academy of Sciences
Shanghai 200031 (CN)**

(72) Inventors:
• **SUN, Bing
Shanghai 200031 (CN)**

• **SUN, Xiaoyu
Shanghai 200031 (CN)**
• **YI, Chunyan
Shanghai 200031 (CN)**
• **LING, Zhiyang
Shanghai 200031 (CN)**
• **ZHANG, Yaguang
Shanghai 200031 (CN)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **FULLY HUMAN BROAD-SPECTRUM NEUTRALIZING ANTIBODY 76E1 AGAINST
CORONAVIRUS, AND USE THEREOF**

(57)    Disclosed are a fully human broad-spectrum neutralizing antibody against coronavirus, and the use thereof. Specifically disclosed are a fully human monoclonal antibody against an S2 region of coronavirus S protein, a nucleic acid sequence encoding the antibody, and a preparation method therefor. The antibody can ef- fectively bind to and neutralize a variety of coronaviruses in a broad spectrum manner, and can be used for preventing and treating diseases related to coronavirus infection, such as SARS-CoV-2. Further disclosed is the potential use thereof in vaccine design.

EP 4 282 880 A1

**Description**

**TECHNICAL FIELD**

The present invention relates to the field of medicine, in particular to a fully human broad-spectrum neutralizing antibody against coronavirus 76E1 and use thereof.

**BACKGROUND**

The outbreak of SARS-CoV-2 has once again aroused people's attention and research on coronavirus. Common coronaviruses such as 229E-CoV, OC43-CoV, NL63-CoV cause only minor respiratory diseases. SARS-CoV-2, SARS-CoV and MERS-CoV are highly infectious and pathogenic, which seriously threaten human health and economic and social stability. Up to August 31, 2020, a total of 25.315 million cases had been confirmed, and 846,000 had died worldwide (real-time tracking of novel coronavirus pneumonia on August 31, 2020). Therefore, it is urgent to develop effective methods for prevention and treatment.

Monoclonal antibody clinical intervention is very effective in the prevention and treatment of viral infections, and has been successfully used clinically to prevent respiratory syncytial virus infection. A large number of preclinical and early clinical data show that monoclonal antibodies can effectively prevent and treat infection caused by a variety of viruses. Therefore, the development of preventive and therapeutic neutralizing antibodies is of great value in combating the current novel coronavirus epidemic or future new and sudden coronavirus infection epidemic.

The surface spike protein of coronavirus is an important antigen for inducing neutralizing antibodies, which play a neutralizing role by blocking the binding of S protein to the receptor or inhibiting the fusion of virus and host cell membrane. The S protein consists of two functional domains, S1 and S2. Among them, S1 is responsible for binding to specific receptors on host cells to promote virus infection, and the sequence is highly variable between different coronaviruses. S2 mediates the fusion of virus and cell membrane and is more conserved than S1. At present, a number of laboratories at home and abroad have isolated fully human monoclonal antibodies targeting SARS-CoV-2 spike protein from patients recovering from the novel coronavirus. Most of these antibodies target the receptor binding region (RBD) of S protein and inhibit virus infection of host cells by blocking the interaction between RBD and angiotensin converting enzyme 2 (ACE2) of the host receptor. Due to the differences in RBD of different coronaviruses, these antibodies can only neutralize SARS-CoV-2, and do not have broad-spectrum neutralizing activity against other coronaviruses or only have weak cross-neutralization against SARS-CoV, and cannot cope with new or sudden onsetnovel coronaviruses in the future. Recently, it has been reported in many literatures that SARS-CoV-2 has been mutated all over the world, and some mutations, especially those in RBD, lead to the ineffectiveness of early isolated neutralizing antibodies. The emergence of neutralizing antibody resistance sites targeting RBD limits the range of antibodies that can be used. Therefore, it is particularly important to find broad-spectrum neutralizing antibodies against coronavirus and antibodies against new epitopes. At present, antibodies targeting the conserved region S2 of the novel coronavirus have not been reported, and there is a lack of systematic research.

Therefore, there is still a need in the field to develop more effective human monoclonal antibodies that can prevent and control the infection of novel coronavirus.

**SUMMARY OF THE INVENTION**

The purpose of the present invention is to provide a fully human broad-spectrum neutralizing antibody able to prevent and control coronavirus infection.

In the first aspect of the present invention, it provides a heavy chain variable region of an antibody comprising the following three complementarity determining regions (CDRs):

CDR1 as shown in SEQ ID NO.: 3,
CDR2 as shown in SEQ ID NO.: 4,
CDR3 as shown in SEQ ID NO.: 5.

In another preferred embodiment, any one of the above amino acid sequences further includes a derivative sequence that is optionally added, deleted, modified and/or substituted with at least one (such as 1-3, preferably 1-2, more preferably 1) amino acid and can retain the binding affinity of coronavirus S protein (preferably S2 protein).

In another preferred embodiment, the heavy chain variable region further includes a human derived FR region or a mouse derived FR region.

In another preferred embodiment, the heavy chain variable region has the amino acid sequence as shown in SEQ ID NO.: 1.

In the second aspect of the present invention, it provides a heavy chain of an antibody having the heavy chain variable region of the first aspect of the present invention.

In another preferred embodiment, the heavy chain of the antibody further includes heavy chain constant region.

In another preferred embodiment, the heavy chain constant region is of human origin, murine origin or rabbit origin.

In the third aspect of the present invention, it provides a light chain variable region of an antibody comprising the following three complementarity determining regions (CDRs):

> CDR1' as shown in SEQ ID NO.: 6,
> CDR2' with an amino acid sequence of EVN, and
> CDR3' as shown in SEQ ID NO.: 8.

In another preferred embodiment, any one of the above amino acid sequences further includes a derivative sequence that is optionally added, deleted, modified and/or substituted with at least one (such as 1-3, preferably 1-2, more preferably 1) amino acid and can retain the binding affinity of coronavirus S protein (preferably S2 protein).

In another preferred embodiment, the light chain variable region further includes a human derived FR region or a mouse derived FR region.

In another preferred embodiment, the light chain variable region has the amino acid sequence as shown in SEQ ID NO.: 2.

In the fourth aspect of the present invention, it provided a light chain of an antibody having the light chain variable region of the third aspect of the present invention.

In another preferred embodiment, the light chain of the antibody further includes a light chain constant region.

In another preferred embodiment, the light chain constant region is of human origin, murine origin or rabbit origin.

In the fifth aspect of the present invention, it provides an antibody having:

> (1) the heavy chain variable region of the first aspect of the present invention; and/or
> (2) the light chain variable region of the third aspect of the present invention;

alternatively, the antibody has: the heavy chain of the second aspect of the present invention; and/or the light chain of the fourth aspect of the present invention.

In another preferred embodiment, the antibody is a specific anti-coronavirus antibody, preferably a specific anti-S protein (preferably S2 protein) antibody.

In another preferred embodiment, the antibody is selected from the group consisting of an animal-derived antibody, a chimeric antibody, a humanized antibody, and a combination thereof.

In another preferred embodiment, the antibody is a double-chain antibody or a single-chain antibody.

In another preferred embodiment, the antibody is a monoclonal antibody or a polyclonal antibody.

In another preferred embodiment, the antibody is a partially or fully humanized monoclonal antibody.

In another preferred embodiment, the antibody is in the form of a drug conjugate.

In another preferred embodiment, the heavy chain variable region sequence of the antibody is as shown in SEQ ID NO.: 1; and the light chain variable region sequence of the antibody is as shown in SEQ ID NO.: 2.

In the sixth aspect of the present invention, it provides a recombinant protein having:

> (i) the heavy chain variable region of the first aspect of the present invention, the heavy chain of the second aspect of the present invention, the light chain variable region of the third aspect of the present invention, the light chain of the fourth aspect of the present invention, or the antibody of the fifth aspect of the present invention; and
> (ii) optionally a tag sequence assisting expression and/or purification.

In another preferred embodiment, the tag sequence includes a 6His tag, a GGGS sequence, and an FLAG tag.

In another preferred embodiment, the recombinant protein or polypeptide includes fusion protein.

In another preferred embodiment, the recombinant protein is a monomer, dimer, or polymer.

In another preferred embodiment, the recombinant protein specifically binds to the coronavirus S protein, preferably to the S2 protein, more preferably to the peptide at positions 809-823 of S protein, and most preferably to the peptide at positions 809-833 of S protein.

In the seventh aspect of the present invention, it provides a CAR construct, wherein the scFv of the antigen binding region of the CAR construct specifically binds to binding region of coronavirus S protein (preferably S2 protein), and the scFv has the heavy chain variable region of the first aspect of the present invention and the light chain variable region of the third aspect of the present invention.

In the eighth aspect of the present invention, it provides a recombinant immune cell, which expresses exogenous CAR of the seventh aspect of the present invention.

In another preferred embodiment, the immune cell is selected from the group consisting of a NK cell, a T cell.

In another preferred embodiment, the immune cells are derived from a human or non-human mammal, such as a mouse.

In the ninth aspect of the present invention, it provides an antibody drug conjugate comprising:

(a) an antibody moiety selected from the group consisting of the heavy chain variable region of the first aspect of the present invention, the heavy chain of the second aspect of the present invention, the light chain variable region of the third aspect of the present invention, the light chain of the fourth aspect of the present invention, or the antibody of the fifth aspect of the present invention, and a combination thereof; and

(b) a coupling moiety conjugated to the antibody moiety, wherein the coupling moiety is selected from the group consisting of a detectable label, a drug, a toxin, a cytokine, a radionuclide, an enzyme, and a combination thereof.

In another preferred embodiment, the coupling moiety is selected from the group consisting of: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), a chemotherapeutic agent (e.g., cisplatin) or a nanoparticle in any form, etc.

In another preferred embodiment, the antibody moiety is coupled with the coupling moiety by chemical bonds or linkers.

In the tenth aspect of the present invention, it provides a use of an active ingredient, wherein the active ingredient is selected from the group consisting of the heavy chain variable region of the first aspect of the present invention, the heavy chain of the second aspect of the present invention, the light chain variable region of the third aspect of the present invention, the light chain of the fourth aspect of the present invention, the antibody according to the fifth aspect of the present invention, the recombinant protein of the sixth aspect of the present invention, and a combination thereof, and the active ingredient is used for the preparation of a medicament, a reagent, a detection plate or a kit.

In another preferred embodiment, the reagent, detection plate or kit is used for detecting coronavirus.

In another preferred embodiment, the medicament is for treating or preventing coronavirus infection.

In another preferred embodiment, the reagent comprises a chip and an antibody-coated immunoparticle.

In the eleventh aspect of the present invention, it provides a pharmaceutical composition containing:

(i) an active ingredient selected from the group consisting of the heavy chain variable region of the first aspect of the present invention, the heavy chain of the second aspect of the present invention, the light chain variable region of the third aspect of the present invention, the light chain of the fourth aspect of the present invention, the antibody according to the fifth aspect of the present invention, the recombinant protein of the sixth aspect of the present invention, the immune cell of the eighth aspect of the present invention, the antibody drug conjugate of the ninth aspect of the present invention, and a combination thereof, and

(ii) a pharmaceutically acceptable carrier.

In another preferred embodiment, the pharmaceutical composition is a liquid preparation.

In another preferred embodiment, the pharmaceutical composition is an injection.

In another preferred embodiment, the pharmaceutical composition is used for preventing and/or treating coronavirus infection.

In the twelfth aspect of the present invention, it provides a polynucleotide encoding a polypeptide selected from the group consisting of:

(1) the heavy chain variable region of the first aspect of the present invention, the heavy chain of the second aspect of the present invention, the light chain variable region of the third aspect of the present invention, the light chain of the fourth aspect of the present invention, or the antibody of the fifth aspect of the present invention; or

(2) the recombinant protein of the sixth aspect of the present invention.

(3) the CAR construct of the seventh aspect of the present invention.

In another preferred embodiment, the polynucleotide has a sequence as shown in SEQ ID NO.: 8 and/or SEQ ID NO.: 9.

In the thirteenth aspect of the present invention, it provides a vector comprising the polynucleotide of the twelfth aspect of the present invention.

In another preferred embodiment, the vector includes: a bacterial plasmid, a bacteriophage, a yeast plasmid, a plant cell virus, a mammalian cell virus such as an adenovirus, a retrovirus, or other vectors.

In the fourteenth aspect of the present invention, it provides a host cell comprising the vector of the thirteenth aspect of the present invention, or having the polynucleotide of the twelfth aspect of the present invention integrated in the genome.

In the fifteenth aspect of the present invention, it provides a method for detecting coronavirus in a sample, which comprises the steps:

(1) contacting the sample with the antibody of the fifth aspect of the present invention;
(2) detecting the formation of an antigen-antibody complex, wherein the formation of a complex indicates the presence of coronavirus in the sample.

In another preferred embodiment, the detection is for non-therapeutic and non-diagnostic purposes.

The present invention also provides a method for detecting coronavirus S protein in a sample, which comprises the steps:

(1) contacting the sample with the antibody of the fifth aspect of the present invention;
(2) detecting the formation of an antigen-antibody complex, wherein the formation of a complex indicates the presence of coronavirus S protein in the sample.

In another preferred embodiment, the coronavirus S protein is coronavirus S2 protein.

In another preferred embodiment, the detection is for non-therapeutic and non-diagnostic purposes.

In the sixteenth aspect of the present invention, it provides a detection plate comprising: a substrate(support plate) and a test strip, wherein the test strip containing the antibody of fifth aspect of the present invention, or the immunoconjugate of the ninth aspect of the present invention.

In the seventeenth aspect of the present invention, it provides a kit comprising:

(1) the first container containing the antibody of the fifth aspect of the present invention; and/or
(2) the second container containing the secondary antibody against the antibody of the fifth aspect of the present invention;

alternatively, the kit comprises the detection plate of the sixteenth aspect of the present invention.

In the eighteenth aspect of the present invention, it provides a method for preparing a recombinant polypeptide, which comprises the steps:

(a) under conditions suitable for expression, culturing the host cell of the fourteenth aspect of the present invention;
(b) isolating the recombinant polypeptide from the culture, wherein the recombinant polypeptide is the antibody of the fifth aspect of the present invention or the recombinant protein of the sixth aspect of the invention.

In the nineteenth aspect of the present invention, it provides a method of treating coronavirus infection which comprises: administering the antibody of the fifth aspect of the present invention, the antibody-drug conjugate thereof, or the CAR-T cell expressing the antibody, and a combination thereof to a subject in need of.

In the twentieth aspect of the present invention, it provides a vaccine composition comprising:

(I) a polypeptide comprising the amino acid sequence of positions 809-833 of the SARS-CoV-2 S2 protein as shown in SEQ ID NO.:10 or a partial sequence thereof, wherein the partial sequence comprises the amino acid sequence of positions 809-823 of the SARS-CoV-2 S2 protein as shown in SEQ ID NO.: 1 1; and
(ii) a vaccine acceptable carrier.

In another preferred embodiment, the identity of the polypeptide with the amino acid sequence shown in SEQ ID NO.: 10 is ≥ 85%, preferably ≥ 90%, and more preferably ≥ 95%.

In another preferred embodiment, the identity of the polypeptide with the amino acid sequence shown in SEQ ID NO.: 10 is ≥ 85%, >_ 86%, >_ 87%, >_ 88%, >_ 89%, >_ 90%, > 91%, ≥ 92%, ≥ 93%, ≥ 94%, ≥ 95%, ≥ 96%, ≥ 97%, ≥ 98%, or ≥ 99%.

In another preferred embodiment, the carrier is a pharmaceutically acceptable carrier.

In another preferred embodiment, the pharmaceutically acceptable carrier contains liquid, preferably water, saline or buffer.

In another preferred embodiment, the carrier further contains an auxiliary substance, preferably a filler, a lubricant, a glidant, a wetting agent or an emulsifier, a pH buffer substance, etc.

In another preferred embodiment, the carrier further contains a cell transfection agent.

In another preferred embodiment, the vaccine composition is a dual vaccine or a multiple vaccine.

In another preferred embodiment, the vaccine composition is a vaccine for preventing novel coronavirus infection.

In another preferred embodiment, the vaccine composition may also contain one or more vaccine components derived from pathogens selected from the group consisting of: SARS-CoV, MERS-CoV, HcoV-229E, HcoV-OC43, HcoV-NL63, HCoV-HKU1, and a combination thereof.

In another preferred embodiment, the vaccine component includes an inactivated strain, an attenuated strain, or a protein, a polypeptide, a nucleic acid, or the like.

In another preferred embodiment, the vaccine composition further comprises an adjuvant.

In another preferred embodiment, the adjuvant comprises a granular and non-granular adjuvant.

In another preferred embodiment, the granular adjuvant is selected from the group consisting of: aluminum salt, water-in-oil emulsion, oil-in-water emulsion, nanoparticle, micro-particle, liposome, immunostimulatory complex, and a combination thereof.

In another preferred embodiment, the non-granular adjuvant is selected from the group consisting of muramyl dipeptide and its derivative, saponin, lipid A, cytokine, derived polysaccharide, bacterial toxin, microorganism and their product such as mycobacteria (Mycobacterium tuberculosis, BCG vaccine), Bacillus pumilus, Bacillus pertussis, propolis, and a combination thereof.

In another preferred embodiment, the vaccine composition is in the form of injection.

In another preferred embodiment, the vaccine composition is a recombinant subunit vaccine, a vector vaccine, a synthetic peptide vaccine, a nucleic acid vaccine, and a combination thereof.

In the twenty-first aspect of the present invention, it provides an inhibitor, wherein the inhibitor targets SARS-CoV-2 S2 protein 809-823 or SARS-CoV-2 S2 protein 809-833 linear epitope for inhibiting novel coronavirus infection.

In another preferred embodiment, the inhibitor is (a) a polypeptide comprising the amino acid sequence of positions 809-833 of the SARS-CoV-2 S2 protein as shown in SEQ ID NO.: 10 or a partial sequence thereof, wherein the partial sequence comprises the amino acid sequence of positions 809-823 of the SARS-CoV-2 S2 protein as shown in SEQ ID NO.:11.

In another preferred embodiment, the inhibitor is a polypeptide comprising the amino acid sequence of positions 809-823 of the SARS-CoV-2 S2 protein as shown in SEQ ID NO.:10.

In another preferred embodiment, the inhibitor is a polypeptide comprising the amino acid sequence of positions 809-823 of the SARS-CoV-2 S2 protein as shown in SEQ ID NO.:11.

In another preferred embodiment, the identity of the polypeptide with the amino acid sequence shown in SEQ ID NO.: 10 or 11 is ≥ 85%, preferably ≥ 90%, and more preferably ≥ 95%.

In another preferred embodiment, the identity of the polypeptide with the amino acid sequence shown in SEQ ID NO.: 10 or 11 is ≥ 85%, ≥ 86%, ≥ 87%, ≥ 88%, ≥ 89%, ≥ 90%, ≥ 91%, ≥ 92%, ≥ 93%, ≥ 94%, ≥ 95%, ≥ 96%, ≥ 97%, ≥ 98%, or ≥ 99%.

In another preferred embodiment, the inhibitor is a polypeptide as shown in SEQ ID NO.:10 or 11.

In another preferred embodiment, the inhibitor is (b) a small molecule compound targeting SARS-CoV-2 S2 protein 809-823 linear epitope or SARS-CoV-2 S2 protein 809-833 linear epitope.

In another preferred embodiment, the inhibitor is a combination of (a) and (b).

It should be understood that within the scope of the present invention, each technical features of the present invention described above and in the following (such as examples) may be combined with each other to form a new or preferred technical solution, which is not listed here due to space limitations.

## DESCRIPTION OF THE DRAWINGS

Figure 1 shows the binding activity of 76E1 antibody with full-length S protein, S1 protein and S2 protein of coronavirus SARS-CoV-2. Among them, 76E1 antibody can bind to full-length S protein and S2 protein of SARS-CoV-2, but cannot bind to S1 protein.

Figure 2 shows that 76E1 antibody can broadly bind to the S protein of different coronaviruses. Among them, Figures 2A-C show the binding of 76E1/76F6/28-12 to the S protein of 7 strains of coronaviruses that infect humans; Figure 2D shows the affinity analysis results of S proteins of 7 strains of *in vitro* purified coronaviruses with 76E1; Figures 2E-G show the flow cytometry analysis results of the binding activity of 76E1 with S protein on the cell surface; Figures 2H-I show that 76E1 antibody can broadly bind to different novel coronavirus mutant strains.

Figure 3 shows that 76E1 antibody can broadly neutralize different coronaviruses and novel coronavirus mutant pseudoviruses.

Figure 4 shows that 76E1 antibody can neutralize SARS-CoV-2 euvirus.

Figure 5 shows that 76E1 antibody recognizes the 809-833 and 809-823 linear epitopes of S2.

Figure 6 shows the results of crystal violet staining of 76E1 antibody inhibiting the fusion of virus with cell membrane.

Figure 7 shows the western blot results of inhibition of cleavage at the S2' cleavage site by 76E1 antibody.

Figure 8 shows the 76E1 antibody animal protection protocol.

Figure 9 shows that 76E1 antibody protects ACE2 humanized mice from being infected with SARS-CoV-2 euvirus, and reduces their weight loss, and reduces the viral load in the lungs of ACE2 humanized mice after being infected with SARS-CoV-2 euvirus.

## DETAILED DESCRIPTION

Through extensive and in-depth research, the present inventors unexpectedly obtained a strain of fully human monoclonal antibody 76E1 against coronavirus S2 protein. The antibody can bind to coronavirus S2 proteins with broad-spectrum, and has high binding and neutralizing activity to coronaviruses, with broad-spectrum recognition and broad-spectrum neutralization, can inhibit or prevent coronaviruses from infecting susceptible cells. *In vivo* and *in vitro* experiments have confirmed that 76E1 antibody can effectively prevent and control coronavirus infection. On this basis, the present invention has been completed.

In the present invention, a strain of fully broad-spectrum neutralizing antibody 76E1 against coronavirus is screened from PBMCs of a volunteer patient recovering from novel coronavirus infection through single cell RT-PCR technology. ELISA binding experiments and cell-level neutralization experiments have confirmed 76E1 antibody has broad-spectrum binding activity and broad-spectrum neutralization activity against the coronavirus S2 protein. 76E1 antibody is a fully human antibody and does not contain murine components, which means that it has lower immunogenicity and higher safety, which indicates the potential clinical application value of coronavirus infection, to provide a new type of candidate drug for coronavirus infection. The antibody of the present invention binds to coronavirus S protein, especially the fusion peptide at positions 809-823 of S protein or fusion peptide at positions 809-833 of S protein, and the exploration of the epitopes of 76E1 antibody also provides some new ideas and references for the design of coronavirus vaccines.

### Term

In order to make this disclosure easier to understand, certain technical and scientific terms are specifically defined below. Unless specifically defined otherwise herein, all other technical and scientific terms used herein have the meaning commonly understood by those skilled in the art to which the present invention belongs. Before the present invention is described, it should be understood that it is not limited to particular methods and experimental conditions described, as such methods and conditions may vary. It should also to be understood that the terms used herein is for the purpose of describing particular embodiments only and is not intended to be limiting, the scope of the present invention will be limited only by the appended claims.

Unless otherwise defined, all technical and scientific terms used herein have the same meanings as those commonly understood by those skilled in the art to which the present invention belongs. As used herein, when used in reference to specific enumerated values, the term "about" means that the value may vary by no more than 1% from the enumerated value. For example, as used herein, "about 100" includes all values between 99 and 101 (e.g., 99.1, 99.2, 99.3, 99.4, etc.).

The amino acid three-letter code and one-letter code used in the present invention are as described in J.biol.chem,243,p3558(1968).

As used herein, the term "treatment" refers to the administration of an internal or external therapeutic agent comprising the monoclonal antibodies against coronavirus S protein (preferably S2 protein) and a composition thereof of the present invention to a patient having one or more disease symptoms for which the therapeutic agent is known to have a therapeutic effect. Generally, the amount of a therapeutic agent that effectively relieves one or more disease symptoms (therapeutically effective amount) is administrated to a patient.

As used herein, the terms "optional" or "optionally" mean that the subsequently described event or circumstance may occur but not necessarily. For example, "optionally contains 1-3 antibody heavy chain variable regions" refers to the specific sequence of the antibody heavy chain variable region can have, but not necessarily, may be 1, 2 or 3.

"Sequence identity" described in the present invention indicates the degree of identity between two nucleic acids or two amino acid sequences when optimally aligned and compared with appropriate mutations such as substitutions, additions or deletions. The sequence identity between the sequence described in the present invention and the sequence with identity to it can be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, 100%.

**Coronavirus (CoV)**

The coronavirus belongs to the order of Nidovirales, the Coronaviridae, and the Coronavirus genus in systematic classification. Coronavirus is a kind of RNA virus with envelope and linear single strand genome. It is a kind of virus widely existing in nature. Coronavirus has a diameter of about 80-120 nm, a cap-like structure with methylation at the 5' end of the genome, a poly (A) tail at the 3' end, and a total genome length of about 27-32kb, which is the largest virus in the known RNA virus. It only infects vertebrates, such as humans, mice, pigs, cats, dogs, wolves, chickens, cattle, birds.

The 2019 novel coronavirus (SARS-CoV-2, causing the novel coronavirus pneumonia COVID-19) is the seventh known coronavirus that can infect humans. The remaining six are HCoV-229E, HCoV-OC43, HCoV-NL63, HCoV-HKU1, SARS-CoV (causing severe acute respiratory syndrome) and MERS-CoV (causing Middle East respiratory syndrome).

For further access to better efficacy, new coronavirus antibody drugs and the search for new antibody recognition epitopes, a broad-spectrum neutralizing antibody 76E1 was isolated from human peripheral blood PBMCs by single cell RT-PCR technology in the present invention. On the one hand, the discovery of new antibodies provides a new choice for the therapeutic application of broad-spectrum neutralizing antibodies, on the other hand, the discovery of new epitopes provides new ideas for the development of broad-spectrum vaccines.

**Antibody**

As used herein, the term "antibody" or "immunoglobulin" is a heterotetrameric glycoprotein of about 150,000 Da having the same structural characteristics, which consists of two identical light chains (L) and two identical heavy chains (H). Each light chain is linked to a heavy chain via a covalent disulfide bond, and different immunoglobulin isotypes have different numbers of disulfide bonds between the heavy chains. There are also regularly spaced intrachain disulfide bonds in each heavy and each light chain. Each heavy chain has a variable region (VH) at one end, followed by a plurality of constant regions. Each light chain has a variable region (VL) at one end and a constant region at the other end; the constant region of light chain pairs with the first constant region of heavy chain, and the variable region of light chain pairs with the variable region of heavy chain. Special amino acid residues form an interface between the variable regions of a light chain and a heavy chain.

As used herein, the term "variable" means that certain portion of the variable region in an antibody differ in sequence, which is responsible for the binding and specificity of various specific antibodies to their specific antigen. However, the variability is not distributed evenly throughout the variable regions of an antibody. It is concentrated in three fragments called complementarity determination regions (CDRs) or hypervariable regions in light chain and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partical β-sheet structure. The CDRs in each chain are closely linked together via the FR regions, and together with the CDRs of the other chain, form the antigen binding site of an antibody (see Kabat et al., NIH Publ. No. 91-3242, Volume I, pages 647-669 (1991)). Constant regions are not directly involved in the binding of antibodies to antigen, however, they exhibit different effector functions, such as participating in the antibody-dependent cytotoxicity of antibodies.

The "light chain" of vertebrate antibodies (immunoglobulins) can be classified into one of two significantly different categories (called κ and λ) according to the amino acid sequence of its constant region. According to the amino acid sequence of its heavy chain constant region, immunoglobulins can be divided into different types. There are mainly 5 types of immunoglobulins: IgA, IgD, IgE, IgG and IgM, some of which can be further divided into subtypes (isotype), such as IgG1, IgG2, IgG3, IgG4, IgA and IgA2. The heavy chain constant regions corresponding to different types of immunoglobulins are called α, δ, ε, γ, and μ. The subunit structure and three-dimensional configuration of different classes of immunoglobulins are well known to those in the art.

As used herein, the term "monoclonal antibody (mAb)" refers to an antibody obtained from a substantially homogeneous population, i.e., the individual antibodies contained in the population are the same, except for a few naturally occurring mutations that may be present. A monoclonal antibody is highly specific for a single antigenic site. Moreover, unlike conventional polyclonal antibody preparations (usually with different antibodies directed against different determinants), each monoclonal antibody is directed against a single determinant on the antigen. In addition to their specificity, the advantage of monoclonal antibodies is that they can be synthesized by hybridoma culture and will not be contaminated by other immunoglobulins. The modifier "monoclonal" indicates the characteristics of antibodies, which are obtained from a substantially uniform antibody population, which should not be interpreted as requiring any special method to produce antibodies.

The present invention also includes monoclonal antibodies with corresponding amino acid sequences of monoclonal antibodies against coronavirus S protein (preferably S2 protein), monoclonal antibodies with variable region chains of monoclonal antibodies against coronavirus S protein (preferably S2 protein), as well as other proteins or protein conjugates and fusion expression products with these chains. Specifically, the present invention includes any protein or protein conjugate and fusion expression product (i.e., immunoconjugate and fusion expression product) having a heavy chain and a light chain containing hypervariable regions, as long as the hypervariable region is the same as or has at least 90% homology with the hypervariable regions of the heavy chain and light chain of the antibody of the present invention, preferably at least 95% homology.

As known to those skilled in the art, an immunoconjugate and the fusion expression product includes: a conjugate formed by a drug, a toxin, a cytokine, a radionuclide, an enzyme and other diagnostic or therapeutic molecules binding to the anti-coronavirus S protein monoclonal antibody or a fragment thereof of the present invention. The present invention also includes a cell surface marker or antigen that binds to the anti-coronavirus S protein monoclonal antibody or a fragment thereof.

The term "antigen-binding fragment of an antibody" (or abbreviated "antibody fragment") refers to one or more fragments of an antibody that retain the ability to specifically bind to an antigen. It has been shown that fragments of a full-length antibody can be used to perform the antigen binding function of the antibody. Examples of binding fragments included in the term "antigen-binding fragment of an antibody" include (i) an Fab fragment, a monovalent fragment composed of VL, VH, CL, and CH1 domains; (ii) an F(ab')$_2$ fragment, a divalent fragment containing two Fab fragments connected by a disulfide bridge between hinge regions; (iii) an Fd fragment composed of VH and CH1 domains; (iv) an Fv fragment composed of the VH and VL domains of the single arm of an antibody. Fv antibody contains the antibody heavy chain variable region and light chain variable region, but no constant region, and has the smallest antibody fragment of all antigen binding sites. In general, Fv antibody further contains a polypeptide linker between the VH and VL domains and is capable of forming the structure required for antigen binding.

The present invention includes not only complete monoclonal antibodies, but also antibody fragments with immune activity, such as Fab or F(ab')$_2$ fragments; antibody heavy chains; antibody light chains.

The term "epitope" or "antigenic determinant" refers to the specific binding site of immunoglobulin or antibody on the antigen. The epitope usually includes at least 3, 4, 5, 6, 7, 8, 9, 10, 11, 12, 13, 14 or 15 contiguous or non-contiguous amino acids in a unique spatial conformation.

The terms "specific binding", "selective binding", "selectively binding" and "specifically binding" refer to the binding of an antibody to a predetermined epitope on an antigen. Typically, antibodies bind with an affinity (KD) of approximately less than $10^{-7}$ M, such as less than $10^{-8}$ M, $10^{-9}$ M, or $10^{-10}$ M or less.

As used herein, the term "antigenic determinant" refers to a three-dimensional spatial site on an antigen that is not contiguous and is recognized by an antibody or antigen-binding fragment of the present invention.

The present invention includes not only intact antibodies, but also fragments of immunologically active antibodies or fusion proteins formed by antibodies with other sequences. Thus, the present invention also includes fragments, derivatives and analogs of the antibody.

In the present invention, the antibodies include murine, chimeric, humanized or full-human antibodies prepared with techniques well known to those skilled in the art. Recombinant antibodies, such as chimeric and humanized monoclonal antibodies, including human and non-human parts, can be prepared using DNA recombinant techniques well known in the art. The term "murine antibody" in the present invention refers to a monoclonal antibody prepared based on knowledge and skills in the art, targeting the coronavirus S protein. The term "chimeric antibody" refers to an antibody in which a variable region of a murine antibody is fused with a constant region of a human antibody, and the immune response induced by the murine antibody can be reduced. The term "humanized antibody", also known as CDR-grafted antibody, refers to the antibody produced by the transplantation of the CDR sequences of mouse into the variable region frameworks of human antibodies, i.e., different types of human germline antibody framework sequences. Humanized antibodies can overcome the heterologous response induced by chimeric antibodies carring a large number of murine protein components. Such framework sequences can be derived from public DNA databases or published references including germline antibody gene sequences. In order to avoid the decrease of immunogenicity resulting in the decrease of activity, the human antibody variable region framework sequence can be subjected to the least reverse mutation or back mutation to maintain the activity.

In the present invention, the antibody may be monospecific, bispecific, trispecific, or more multiple specificities.

As used herein, the terms "heavy chain variable region" and "VH" can be used interchangeably.

As used herein, the terms "variable region" and "complementarity determine region (CDR)" can be used interchangeably.

The term "CDR" refers to one of the six highly variable regions within the variable domain of an antibody that primarily facilitate the binding to antigen. One of the most commonly used definitions of the six CDRs is provided by Kabat E.A et al., (1991) Sequences of proteins of immunological interest NIH Publication91-3242).

In a preferred embodiment of the present invention, the heavy chain variable region of the antibody comprises the following three complementarity determining regions (CDRs):

CDR1: GFSFKDYG (SEQ ID NO.: 3),
CDR2: ISGDTRGT (SEQ ID NO.: 4), and

CDR3: AALVIVAAGDDFDL (SEQ ID NO.: 5).

In another preferred embodiment, the amino acid sequence of the heavy chain variable region is as shown in SEQ ID NO.: 1, where the underlined sequences are the amino acid sequences of the heavy chain variable region CDR1, CDR2, and CDR3.

EVQLVESGGGVVQPGGSLRLSCEAS<u>GFSFKDYG</u>MHWIRQTPGKGLEWISR<u>ISGDT
RGT</u>SYVDSVKGRFIVSRDNSRNSLFLQMNSLRSEDTALYYC<u>AALVIVAAGDDFDL</u>WG
QGTVVTVSS (SEQ ID NO.: 1)

In another preferred embodiment, the nucleic acid coding sequence of the heavy chain variable region is as shown in SEQ ID NO.: 8, where the underlined sequences are the nucleic acid coding sequences of the heavy chain variable region CDR1, CDR2, and CDR3.

GAGGTGCAGCTGGTGGAGTCTGGGGGAGGCGTGGTGCAGCCGGGGGGGTCCC
TGAGGCTCTCCTGTGAAGCCTCT<u>GGATTCAGCTTTAAAGACTATGGC</u>ATGCACTGG
ATCCGTCAGACTCCAGGGAAGGGTCTGGAGTGGATCTCTCGT<u>ATTAGTGGAGACAC
TAGAGGCACA</u>TCCTATGTAGACTCTGTGAAGGGCCGATTCATCGTCTCCAGAGACA
ACAGCAGAACTCCTTGTTTTTACAAATGAACAGTCTGAGAAGTGAAGACACCGC
CTTGTATTACTGT<u>GCAGCATTAGTTATTGTAGCTGCCGGCGATGATTTTGATCTC</u>TG
GGGCCAAGGGACAGTGGTCACCGTTCTTCA (SEQ ID NO.: 8)

In a preferred embodiment of the present invention, the heavy chain of the antibody comprises the aforementioned heavy chain variable region and heavy chain constant region, which may be of mouse origin or human origin.
As used herein, the terms "light chain variable region" and "V$_L$" can be used interchangeably.
In a preferred embodiment of the present invention, the light chain variable region of the antibody of the present invention has complementarity determining regions (CDRs) selected from the group consisting of:

CDR1': SSDIGSYNF (SEQ ID NO .: 6),
CDR2': EVN, and
CDR3': CSYGGRNNLI (SEQ ID NO.: 7).

In another preferred embodiment, the amino acid sequence of the light chain variable region is as shown in SEQ ID NO.: 2, where the underlined sequences are the amino acid sequences of the light chain variable region CDR1', CDR2', and CDR3'.

QSALTQPLSVSGSPGQSVTISCTGS<u>SSDIGSYNF</u>VSWYRQYPGKAPKVMIY<u>EVN</u>K
RPSGVPVRFSGSKSGNTASLTVSGLQHEDEADYYC<u>CSYGGRNNLI</u>FGGGTKLTVL

(SEQ ID NO.: 2)

In another preferred embodiment, the nucleic acid coding sequence of the light chain variable region is as shown in SEQ ID NO.: 9, where the underlined sequences are the nucleic acid coding sequences of the light chain variable region CDR1', CDR2', and CDR3'.

CAGTCTGCCCTGACTCAGCCTCTCTCAGTGTCCGGGTCTCCTGGACAGTCCGT
CACCATCTCCTGCACTGGATCC<u>AGCAGTGACATTGGGAGTTATAATTTT</u>GTCTCCTG
GTATCGACAATATCCAGGCAAAGCCCCCAAAGTCATGATCTAT<u>GAGGTCAAT</u>AAG
CGGCCCTCGGGGGTCCCTGTTCGCTTCTCTGGCTCCAAGTCTGGCAACACGGCCTC
CCTGACCGTCTCTGGGCTCCAACATGAGGATGAGGCTGACTATTACTGC<u>TGCTCAT</u>
<u>ATGGAGGCCGCAACAATTTGATTT</u>TCGGCGGAGGGACCAAGCTGACCGTCCTA
(SEQ ID NO.: 9)

In a preferred embodiment of the present invention, the light chain of the antibody comprises the aforementioned light chain variable region and heavy chain constant region, which may be of mouse origin or human origin.

The function of the antibody of the present invention is determined by the specific gene sequences of the variable regions of the light and heavy chains of the antibody, which can bind to the S2 protein of the coronavirus in a broad-spectrum manner and prevent the coronavirus from infecting susceptible cells. Using the variable region gene or complementarity-determining region (CDR) gene of the antibody, different forms of genetically engineered antibodies can be modified and produced in any expression system using prokaryotic and eukaryotic cells.

In the present invention, the terms "the antibody of the present invention", "the protein of the present invention", or "the polypeptide of the present invention" can be used interchangeably, both refer to antibodies specifically binding to coronavirus S protein (preferably S2 protein), e.g., a protein or polypeptide with a heavy chain variable region (such as the amino acid sequence encoded by the nucleotide sequence as shown in SEQ ID NO.: 8) and/or a light chain variable region (such as the amino acid sequence encoded by the nucleotide sequence as shown in SEQ ID NO.: 9). They can contain or do not contain starting methionine.

In another preferred embodiment, the antibody is a mouse or human mouse chimeric monoclonal antibody against coronavirus S protein (preferably S2 protein), and its heavy chain constant region and/or light chain constant region can be humanized heavy chain constant region or light chain constant region. More preferably, the humanized heavy chain constant region or light chain constant region is the heavy chain constant region or light chain constant region of human IgG1, IgG2, etc.

In general, the antigen binding characteristics of an antibody can be described by three specific regions located in the heavy chain and light chain variable regions, called the variable regions (CDRs), which separate this segment into four frame regions (FRs). The amino acid sequences of the four FRs are relatively conservative and do not directly participate in the binding reaction. These CDRs form a loop structure, and the β-sheets formed by the FRs in between are spatially close to each other, and the CDRs on the heavy chain and the CDRs on the corresponding light chain constitute the antigen-binding site of the antibody. It can be determined which amino acids constitute the FR or CDR region by comparing the amino acid sequences of antibodies of the same type.

The variable regions of the heavy chain and/or the light chain of the antibody of the present invention are of particular interest because at least part of them involves binding antigens. Therefore, the present invention includes molecules with light and heavy chain variable regions of monoclonal antibodies with CDRs, as long as their CDR has more than 90% (preferably more than 95%, most preferably more than 98%) homology with the CDR identified here.

The present invention includes not only intact monoclonal antibodies, but also fragments of immunologically active antibodies or fusion proteins formed by antibodies with other sequences. Thus, the present invention also includes fragments, derivatives and analogs of the antibody. For example, modification is preformed on the Fc fragment based on the antibody of the present invention, three mutation sites M252Y /S254T /T256E are introduced into the CH2 region in order to prolong the half-life of the antibody.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope of well-known to those skilled in the art.

The antibody of the present invention refers to a polypeptide having coronavirus S protein (preferably S2 protein) binding activity and comprising the above-mentioned CDR regions. The term also includes variant forms of polypeptides comprising the CDR regions described above that have the same function as the antibody of the present invention. These variants include (but are not limited to): deletion, insertion and/or substitution of one or more (usually 1-50, preferably 1-30, more preferably 1-20, most preferably 1-10) amino acids, and addition of one or more (usually within 20, preferably within 10, more preferably within 5) amino acids at the C-terminus and/or N-terminus. For example, in the art, substitutions with amino acids of similar properties generally do not alter the function of the protein. For another example, addition of one or more amino acids to the C-terminus and/or N-terminus usually does not alter the function of the protein. The term also includes active fragments and active derivatives of the antibody of the present invention.

The variant forms of the polypeptide include homologous sequences, conservative variants, alleles, natural mutants, induced mutants, proteins encoded by DNA capable of hybridizing with the coding DNA of the antibody of the present invention under high or low tightness conditions, and polypeptides or proteins obtained by using anti-serum against the antibody of the present invention.

The present invention also provides other polypeptides, such as fusion proteins containing human antibodies or fragments thereof. In addition to the almost full-length polypeptide, the present invention also includes fragments of the human antibody of the present invention. Typically, the fragment has at least about 50 contiguous amino acids, preferably at least about 60 contiguous amino acids, more preferably at least about 80 contiguous amino acids, and most preferably at least about 100 contiguous amino acids of the antibody of the present invention.

In the present invention, "the conservative variant of the antibody of the invention" means that compared with the amino acid sequence of the antibody of the present invention, there are at most 10, preferably at most 8, more preferably at most 5, and preferably at most 3 amino acids are replaced by amino acids with the same or similar properties to form a polypeptide. These conservative variant polypeptides are best produced by amino acid substitution according to Table A.

Table A

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides a polynucleotide molecule encoding the above antibody or fragment thereof or fusion protein thereof. The polynucleotide of the present invention may be in the form of DNA or RNA. DNA form includes cDNA, genomic DNA, or synthetic DNA. DNA may be single-stranded or double-stranded. DNA may be a coding strand or a non-coding strand. The coding region sequence encoding mature polypeptide may be the same as or a degenerate variant of the coding region sequence shown in SEQ ID NO.: 8 or 9. As used herein, "degenerate variant" in the present invention refers to a nucleic acid sequence encoding the same amino acid sequence as the polypeptide of the present invention, but different from the coding region sequence shown in SEQ ID NO.: 8 or 9.

The polynucleotide encoding the mature polypeptide of the present invention includes: the coding sequence that encodes only the mature polypeptide; the coding sequence of the mature polypeptide and various additional coding sequences; the coding sequence of the mature polypeptide (and optionally additional coding sequence) and the non-coding sequence.

The term "polynucleotide encoding a polypeptide" may be a polynucleotide that includes sequence encoding the polypeptide, or a polynucleotide that also includes additional coding and/or non-coding sequences.

The present invention also relates to a polynucleotide that hybridizes to the above-mentioned sequence and has at least 50%, preferably at least 70%, and more preferably at least 80% identity between the two sequences. In particular, the present invention relates to a polynucleotide that is hybridizable to the polynucleotide of the present invention under strict conditions. In the present invention, "strict conditions" refer: (1) hybridization and elution at lower ionic strength and higher temperature, such as $0.2 \times$ SSC, 0.1% SDS, 60°C; or (2) hybridization with denaturing agent, such as 50%(v/v) formamide, 0.1% calf serum/0.1 % Ficoll, 42°C, etc.; or (3) hybridization occurs only when the identity between the two sequences is at least 90% or more, more preferably 95% or more. Furthermore, the polypeptide encoded by the hybridizable polynucleotide has the same biological function and activity as the mature polypeptide shown in SEQ ID NO.: 1 and/or SEQ ID NO.: 2.

The full-length nucleotide sequence or fragments thereof of the antibody of the present invention may generally be obtained by PCR amplification, recombination or artificial synthesis methods. A feasible method is to synthesize the relevant sequence by artificial synthesis, especially when the fragment length is short. Generally, fragments with a long sequence can be obtained by first synthesizing multiple small fragments followed by ligation. In addition, the coding sequence of the heavy chain and the expression tag (such as 6His) can be fused together to form a fusion protein.

Once the relevant sequence is obtained, the recombination method can be used to obtain the relevant sequence in large quantities. This is usually to clone it into a vector, then transfer it into a cell, and then separate the relevant sequence from the proliferated host cell by conventional methods. The biomolecules (nucleic acids, proteins, etc.) involved in the present invention include biomolecules in isolated form.

At present, the DNA sequence encoding the protein (or its fragment, or its derivative) of the present invention can be obtained completely by chemical synthesis. The DNA sequence can then be introduced into various existing DNA molecules (or, for example, vectors) and cells known in the art. In addition, mutations can be introduced into the protein sequence of the present invention by chemical synthesis.

The present invention also relates to a vector comprising the appropriate DNA sequence as described above and an appropriate promoter or control sequence. These vectors can be used to transform appropriate host cells to enable them to express proteins.

Host cells may be prokaryotic cells, such as bacterial cells; or lower eukaryotic cells, such as yeast cells; or higher eukaryotic cells, such as mammalian cells. Representative examples include: Escherichia coli, Streptomyces; bacterial cells of Salmonella typhimurium; fungal cells such as yeast; insect cells of Drosophila S2 or Sf9; animal cells of CHO, COS7, 293 cells, etc.

Transformation of host cells with recombinant DNA can be carried out using conventional techniques well known to those skilled in the art. When the host is a prokaryotic organism such as Escherichia coli, the competent cells capable of absorbing DNA can be harvested after the exponential growth period and treated with $CaCl_2$, the steps used are well known in the art. Another method is to use $MgCl_2$. If necessary, the transformation can also be carried out by electroporation. When the host is eukaryotic, the following DNA transfection methods can be used: calcium phosphate co-precipitation method, conventional mechanical methods such as microinjection, electroporation, liposome packaging, etc.

The obtained transformant can be cultured by conventional methods to express the polypeptide encoded by the gene of the present invention. Depending on the host cell used, the medium used in the culture may be selected from a variety of conventional medium. Culture is carried out under conditions suitable for host cell growth. When the host cells grow to an appropriate cell density, the selected promoter is induced by a suitable method (such as temperature conversion or chemical induction), and the cells are cultured for another period of time.

The recombinant polypeptide in the above method may be expressed in the cell, or on the cell membrane, or secreted outside the cell. If necessary, the recombinant protein can be isolated and purified by various separation methods using its physical, chemical and other properties. These methods are well known to those skilled in the art. Examples of these methods include, but are not limited to, conventional renaturation treatment, treatment with a protein precipitant (salting-out method), centrifugation, osmotic breakage, ultra-treatment, ultra-centrifugation, molecular sieve chromatography (gel filtration), adsorption chromatography, ion exchange chromatography, high performance liquid chromatography (HPLC) and other liquid chromatography techniques and combinations of these methods.

The antibody of the present invention can be used alone, or can be combined or coupled with a detectable label (for diagnostic purposes), a therapeutic agent, a PK (protein kinase) modifying moiety, or any combination of these substances.

A detectable label for diagnostic purposes includes, but is not limited to, a fluorescent or luminescent label, a radioactive label, a MRI (magnetic resonance imaging) or CT (electronic computer tomography) contrast agent, or an enzyme capable of producing a detectable product.

Coupled therapeutic agents include but are not limited to: insulin, IL-2, interferon, calcitonin, GHRH peptide, intestinal peptide analog, albumin, antibody fragments, cytokines, and hormones.

In addition, the therapeutic agents that can be bound or coupled with the antibody of the present invention include, but are not limited to: 1. radionuclides; 2. biotoxins; 3. cytokines such as IL-2 and the like; 4. gold nanoparticles/nanorods; 5. virus particles; 6. liposomes; 7. magnetic nanoparticles; 8. prodrug-activating enzymes; 9. chemotherapeutic agents, (e.g., cisplatin), or nanoparticles in any form, etc.

The present invention also provides a composition. In preferred embodiments, the composition is a pharmaceutical composition comprising the above-mentioned antibody or active fragment thereof or fusion protein thereof, and a pharmaceutically acceptable carrier. Typically, these substances may be formulated in a non-toxic, inert and pharmaceutically acceptable aqueous carrier medium, wherein the pH is typically about 5-8 and preferably about 6-8, although the pH may vary depending on the nature of the substance being formulated and the condition to be treated. The formulated pharmaceutical composition may be administered by conventional routes, including (but not limited to) oral, respiratory, intratumoral, intraperitoneal, intravenous, or topical administration.

The pharmaceutical composition of the present invention can be directly used to bind the coronavirus S protein (preferably S2 protein) molecules, and thus can be used to prolong the half-life of the drug. In addition, other therapeutic agents can also be used.

The pharmaceutical composition of the present invention contains a safe and effective amount (e.g., 0.001-99wt%, preferably 0.01-90wt%, more preferably 0.1-80wt%) of the above-mentioned monoclonal antibody of the present invention (or the conjugate thereof) and a pharmaceutically acceptable carrier or excipient. Such carriers include (but not limited to): saline, buffer, glucose, water, glycerol, ethanol, and combinations thereof. The pharmaceutical formulation should match the mode of administration. The pharmaceutical composition of the present invention may be prepared in the form of an injection, for example, by conventional methods using normal saline or aqueous solutions containing glucose and other adjuvants. The pharmaceutical composition such as an injection and solution should be manufactured under sterile conditions. The dosage of the active ingredient is a therapeutically effective amount, for example, about 1 $\mu$g/kg body weight to about 10 mg/kg body weight per day. In addition, the polypeptide of the present invention may also be used with other therapeutic agents.

When a pharmaceutical composition is used, a safe and effective amount of the immune conjugate is administered to a mammal, wherein the safe and effective amount is typically at least about 10 $\mu$g/kg body weight, and in most cases no more than about 8 mg/kg body weight, preferably about 10 $\mu$g/kg body weight to about 1 mg/kg body weight. Of course, the specific dosage should also be considered with factors such as the administration route and the patient's health status, which are all within the skill range of a skilled physician.

## Detection application and kit

The antibodies of the present invention can be used in detection applications, for example, for detecting a sample to provide diagnostic information.

In the present invention, the sample (specimen) includes a cell sample, a tissue sample, and a biopsy sample. The term 'biopsy' used in the present invention should include all types of biopsies known to those skilled in the art. Therefore, the biopsy used in the present invention can include tissue samples prepared, for example, through endoscopic methods or organ puncture or needle biopsy.

Samples for use in the present invention include fixed or preserved cell or tissue samples.

The present invention also provides a kit containing the antibody (or fragment thereof) of the present invention. In a preferred embodiment of the present invention, the kit further comprises a container, instructions for use, and a buffer, etc. In a preferred embodiment, the antibody of the present invention may be immobilized on a detection plate.

## SARS-CoV-2 S2 protein 809-833 linear epitope

The monoclonal antibody provided by the present invention binds to the SARS-CoV-2 S2 protein 809-823 linear epitope (antigen epitope peptide), preferably the SARS-CoV-2 S2 protein 809-833 linear epitope (antigen epitope peptide), and the specific sequence is as follows:

SARS-CoV-2 S2 protein 809-833 linear epitope (antigen epitope peptide):
PSKPSKRSFIEDLLFNKVTLADAGF (SEQ ID NO.: 10)
SARS-CoV-2 S2 protein 809-823 linear epitope (antigen epitope peptide):
PSKPSKRSFIEDLLF (SEQ ID NO.: 11).

Among them, 815 (R), 819 (E), 823 (F) are the most critical epitopes of the antibody 76E1 of the present invention, and 820 (D) and 822 (L) are the sub-critical epitopes.

Based on these epitopes, the present invention also provides a vaccine composition comprising:

(i) a polypeptide comprising the amino acid sequence of positions 809-833 of the SARS-CoV-2 S2 protein as shown in SEQ ID NO.: 10 or a partial sequence thereof (comprising the amino acid sequence of positions 809-823 of the SARS-CoV-2 S2 protein as shown in SEQ ID NO.: 11); and
(ii) a vaccine acceptable carrier.

In addition, the present invention provides an inhibitor, wherein the inhibitor targets SARS-CoV-2 S2 protein 809-823 linear epitope or SARS-CoV-2 S2 protein 809-833 linear epitope for inhibiting novel coronavirus infection.

In another preferred embodiment, the inhibitor is (a) a polypeptide comprising the amino acid sequence of positions 809-833 of the SARS-CoV-2 S2 protein as shown in SEQ ID NO.: 10 or a partial sequence thereof (comprising the amino acid sequence of positions 809-823 of the SARS-CoV-2 S2 protein as shown in SEQ ID NO.: 11).

In another preferred embodiment, the inhibitor is (b) a small molecule compound targeting SARS-CoV-2 S2 protein 809-833 linear epitope.

In another preferred embodiment, the inhibitor is a combination of (a) and (b).

**The main advantages of the present invention**

(1) The fully human monoclonal antibody of the present invention can specifically recognize and bind to the S protein of the coronavirus, has high neutralizing activity to the coronavirus, and can neutralize 7 strains of coronaviruses infecting humans, including 229E-CoV, OC43-CoV, NL63-CoV, HKU1-CoV, SARS-CoV-2, SARS-CoV and MERS-CoV, and effectively inhibits or prevents coronavirus from infecting susceptible cells.
(2) The fully human monoclonal antibody of the present invention has broad-spectrum binding activity and broad-spectrum neutralizing activity against various coronaviruses, and can effectively neutralize various coronaviruses.
(3) The present invention is about a fully human monoclonal antibody 76E1, which does not contain mouse derived parts and has lower immunogenicity and higher safety for the human body. It can avoid immune rejection reactions mediated by antibodies from other species such as mice.
(4) The fully human monoclonal antibody 76E1 of the present invention binds to the coronavirus S protein, especially the fusion peptide on the S2 protein, which contains the 809-823 amino acids (such as the 809-833 amino acids) of the SARS-CoV-2 S2 protein, which is a linear epitope. The discovery of the epitope for 76E1 antibody also provides some new ideas and references for the design of coronavirus vaccines.

The present invention is further explained below in conjunction with specific examples. It should be understood that these examples are only for illustrating the present invention and do not intend to limit the scope of the present invention. The conditions of the experimental methods not specifically indicated in the following examples are usually in accordance with conventional conditions as described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

Experiments that do not indicate specific conditions in the examples or test examples of the present invention are usually carried out under conventional conditions, or according to the conditions recommended by the raw material/commodity manufacturer. Reagents that are not indicated as from specific sources are commercially available.

The preparation of neutralizing fully human monoclonal antibodies capable of neutralizing coronavirus S protein of the present invention and the analysis of antibody characteristics are described below.

**Example 1 Single-cell RT-PCR assay to obtain antibody genes and antibody expression**

1.1 Obtaining peripheral blood monocytes (PBMCs)

Peripheral blood was drawn from healthy volunteers, and using conventional Ficoll-Paqe (manufacturer: Lympholyte®- H (CEDARLANE)) for density gradient centrifugation to obtain more than $10^7$ peripheral blood monocytes (PBMCs).

Ficoll separation method:

(1) Collecting blood into a 50ml centrifuge tube (pre-contained 4% sodium citrate 1ml), and 20ml of whole blood was collected and mixed upside down 8-10 times. (i.e., the final concentration of sodium citrate was 0.4%);
(2) Equal volume of RPMI1640 (containing sodium citrate) was added and mixed well;
(3) Using a 15ml transparent centrifuge tube, 3ml of lymphocyte separation solution was added, and 6ml of blood sample was carefully added on it. A separation interface was formed (or 4ml of separation solution adding with 8ml of blood sample).
(4) Centrifugation was performed at room temperature at 800g, for 20min (2000rpm, 20min);
(5) The interface layer cells were carefully aspirated and transferred to a new tube;
(6) RPMI1640 (containing sodium citrate) was added for dilution to reduce the density of the liquid. Centrifugation was performed at 800g/2000rpm for 10min. The supernatant was removed;
(7) The cells were washed with RPMI1640 for 2-3 times for later use.

1.2 Obtaining spike protein S-specific memory B cells

Using FITC-CD19/APC-IgG/BV421-S proteins as markers, BD Horizon™ Fixable Viability Stain 780-APC-Cy7 was used to remove dead cells, and CD4/CD14/CD8-percp5.5 was used to remove macrophages and T cells, etc. Specific B cells were obtained by flow cytometry and transferred to a 96-well RT-PCR plate with one cell per well, and the S protein-specific memory B cells were obtained.

(1) SARS-CoV-2 S protein was expressed by mammalian cell CHO expression system. The sequence of the S protein was referenced to nCoV-SH01 (GenBank: MT121215.1), and full gene synthesis was conducted by Shanghai Generay Biotech Co., Ltd;
(2) Biotin-labeled SARS-CoV-2 S protein was used to detect biotin-labeled S protein;
(3) Labeling sorting cells: PBMC cells were divided into experimental group + control group, and labels were added according to the number of cells, staining in the dark for labeling. After being resuspended in PBS, 40 μm BD falcon membrane was used for filtration;
(4) Sorting of specific B cells: Using BD FACS Influx screening, lymphocytes were screened from PBMCs according to the forward angle and lateral angle, and then through the adjustment compensation of different control groups, the specific memory B cells for S protein were obtained, which were sorted into a 96-well plate for RT-PCR (reverse transcription PCR), one cell per well, and the plate was placed on dry ice.

1.3 Antibody gene acquisition and vector construction

The obtained single memory B cell was subjected to RT-PCR to obtain cDNA, and then the antibody variable region gene was obtained by nested-PCR. Running nucleic acid agarose gel electrophoresis and the gel block with paired heavy and light chains was recovered and sequenced. The antibody gene sequences were obtained by searching. Next, the antibody genes were linked to the corresponding IgH, Igκ and Igλ expression vectors. Fully human antibody expression vectors IgH, Igκ and Igλ (expressing antibody heavy chain, kappa chain and lambda chain respectively), were donated by Patrick Wilson laboratory.

1.4 Antibody expression and purification

CHO cells were transiently transfected for fully human antibody expression. One day before transfection (no.-1 day), ExpiCHO-S™ cells were divided and inoculated with a final density of $3 \times 10^6$-$4 \times 10^6$ living cells/mL, and the cells were allowed to grow overnight. Next Day (day 0), the density of viable cells and the percent of survival rate were determined. The cell density should reach about $7 \times 10^6$-$10 \times 10^6$ living cells/mL. The survival rate should be 95-99% in order to continue transfection. The cells were diluted to a final density of $6 \times 10^6$ living cells/mL. Pre-cold reagents (4°C) were used for preparation of Expi Fectamine™ CHO/plasmid DNA complex. Expi Fectamine™ CHO/plasmid DNA complex was incubated at room temperature for 1-5 minutes, then the solution was slowly transferred into the CHO cell culture flask, gently shaking the flask during the addition. The cells were placed on an orbital shaker (under humidified air conditions with 8% $CO_2$ in a 37 °C incubator) for culture. After 7-11 days of culture, when half of the cells were dead, the supernatant could be collected and the antibody could begin to be purified.

Protein G Agarose 4FF filler (purchased from GE) was used to purify the antibody. Firstly, the collected CHO cells suspension was centrifuged at 4000 rpm and 4°C for 30min, then the collected supernatant was filtered by a 0.45um filter for purification. Taking the gravity type centrifugal column and adding the Protein G Agarose 4FF filler, 3-fold column volume of 20% ethanol was used to stabilize the filler, followed by 5-fold column volume of binding buffer being used to equilibrate the column, then the sample was loaded, and then the column was equilibrated with 10-fold column volume of binding buffer, finally the column was eluted with 3-fold column volume of elution buffer. A neutralization buffer was added to the eluted antibody solution to achieve a pH of around 7.5 for the eluted sample. Antibody solution was dialyzed in 5L of 1*PBS for 3 times, and the antibody can be concentrated and stored at -80°C.

1.5 Experimental results:

Single cell RT-PCR and Nested-PCR methods were used to obtain the matching antibody heavy and light chain variable region genes, with a molecular weight of about 400bp. Agarose gel was recovered and sequenced. Sequencing results were compared in http://www.ncbi.nlm.nih.gov/igblast and http://www.imgt.org/, the antibody germline gene information and the antibody heavy or light chain gene high variable region information were obtained, and the expression vector construction and subsequent expression purification were carried out. Finally, through this technology, a fully human monoclonal antibody with broad-spectrum neutralization against coronavirus was successfully obtained, named 76E1.

The heavy chain variable region gene sequence of the fully human antibody 76E1 is as follows, where underlined are the hyper variable region sequences in the heavy chain gene variable region, which are the heavy chain gene CDR1, CDR2 and CDR3 sequences successively.

GAGGTGCAGCTGGTGGAGTCTGGGGGAGGCGTGGTGCAGCCGGGGGGGTCCCT
GAGGCTCTCCTGTGAAGCCTCT<u>GGATTCAGCTTTAAAGACTATGGC</u>ATGCACTGGATC
CGTCAGACTCCAGGGAAGGGTCTGGAGTGGATCTCTCGT<u>ATTAGTGGAGACACTAGA
GGCACA</u>TCCTATGTAGACTCTGTGAAGGGCCGATTCATCGTCTCCAGAGACAACAGCA
GAAACTCCTTGTTTTTACAAATGAACAGTCTGAGAAGTGAAGACACCGCCTTGTATTA
CTGT<u>GCAGCATTAGTTATTGTAGCTGCCGGCGATGATTTTGATCTC</u>TGGGGCCAAGGG
ACAGTGGTCACCGTTTCTTCA (SEQ ID NO.: 8)

The heavy chain variable region amino acid sequence of the fully human antibody 76E1 is as follows, where underlined in order are the heavy chain amino acid CDR1, CDR2, CDR3 sequences.

EVQLVESGGGVVQPGGSLRLSCEAS<u>GFSFKDYG</u>MHWIRQTPGKGLEWISR<u>ISGDTRG
T</u>SYVDSVKGRFIVSRDNSRNSLFLQMNSLRSEDTALYYC<u>AALVIVAAGDDFDL</u>WGQGTV
VTVSS (SEQ ID NO.: 1)

The light chain variable region gene sequence of the fully human antibody 76E1 is as follows, where underlined are the hyper variable region sequences in the light chain gene variable region, which are the light chain gene CDR1', CDR2' and CDR3' sequences successively.

CAGTCTGCCCTGACTCAGCCTCTCTCAGTGTCCGGGTCTCCTGGACAGTCCGTCA
CCATCTCCTGCACTGGATCC<u>AGCAGTGACATTGGGAGTTATAATTT</u>GTCTCCTGGTAT
CGACAATATCCAGGCAAAGCCCCCAAAGTCATGATCTAT<u>GAGGTCAAT</u>AAGCGGCCC
TCGGGGGTCCCTGTTCGCTTCTCTGGCTCCAAGTCTGGCAACACGGCCTCCCTGACCGT
CTCTGGGCTCCAACATGAGGATGAGGCTGACTATTACTGC<u>TGCTCATATGGAGGCCGC
AACAATTTGATT</u>TTCGGCGGAGGGACCAAGCTGACCGTCCTA (SEQ ID NO.: 9)

The light chain variable region amino acid sequence of the fully human antibody 76E1 is as follows, where underlined in order are the light chain amino acid CDR1', CDR2', CDR3' sequences.

QSALTQPLSVSGSPGQSVTISCTGS<u>SSDIGSYNF</u>VSWYRQYPGKAPKVMIY<u>EVN</u>KRPS GVPVRFSGSKSGNTASLTVSGLQHEDEADYYC<u>CSYGGRNNLI</u>FGGGTKLTVL (SEQ ID NO.: 2)

**Example 2 Antibody characteristic analysis**

2.1 ELISA detection of antibody binding activity to antigen

ELISA was used to test whether the expressed antibodies recognized different coronavirus S proteins. S proteins of different coronaviruses were purchased from Beijing Sino Biological Inc.. 28-12 is the negative control antibody, which is produced by the present laboratory. The ELISA plate was coated with S protein, 0.5 $\mu$g/mL, 100 $\mu$L per well, at 4°C overnight. Next day, the plate was washed with PBST for 3 times. 2% BSA was added for blocking, 200 $\mu$L per well, 37°C, 2 h. The plate was washed with PBST for 3 times again. 76E1 and control antibody were diluted by the 3-fold gradient dilution, with an initial test concentration of 10 $\mu$g/ml. Loading the sample, 100 $\mu$L per well, 37°C, 2 h. The plate was washed with PBST for 3 times. Goat Anti-Human IgG (Fc specific)- Peroxidase antibody (sigma), 1:8000 diluted, 100 $\mu$L per well, 37°C, 1 h. The plate was washed with PBST for 3 times. Substrate TMB was added at 100 $\mu$L/well for development. If the color is lighter, it can react in dark at 37°C for 15 minutes. 2M $H_2SO_4$ was added to terminate the reaction, 50 $\mu$L per well. $OD_{450}$ was determined and data processing was performed.

2.2 Bio-Layer Interferometry (BLI) Affinity Detection

This experiment was performed on the molecular platform of the Institute of Biochemical Cell using the OCTET RED 96 instrument. First of all, AHC sensor was soaked in a 96-well black plate with PBS for at least 10min, and 76E1 was added with a concentration of 20ug/ml. Concentration gradient of antigen was set, 2-fold gradient dilution: 200nM-100nM-50nM-25nM-12.5nM-6.25 nM; The antigen of antibody, regeneration buffer and PBS buffer were added to another black plate and placed on the instrument. The program was set and started running. The program is as follows: baseline 120s $\rightarrow$ Ab $\rightarrow$ 300s-baseline $\rightarrow$ 240s-association$\rightarrow$ 900s-disassociation$\rightarrow$ 900s-regeneration $\rightarrow$ 5s-baseline $\rightarrow$ 5s-baseline $\rightarrow$ 5s-regeneration $\rightarrow$ 5s-baseline $\rightarrow$ 5s-baseline $\rightarrow$ 5s. The data was analyzed with software and the KD value of antigen-antibody affinity was calculated.

2.3 Pseudovirus neutralization assay

The S protein plasmids of full-length SARS-CoV, SARS-CoV-2, MERS-CoV and other human coronavirus and pNL4-3 plasmid were used to co-transfect HEK293T cells in a 10cm culture dish, and the medium was changed after 6 hours. The supernatant was collected after 48 hours and diluted with complete medium. Antibodies were serially diluted and mixed with an equal volume of virus and incubated for 1h at 37°C. The mixture of the antibody and virus was transferred into HEK293T cells stably expressing human ACE2 or cells expressing the corresponding coronavirus receptor. Incubation was performed at 37°C for 48 hours. The supernatant was removed, and lysis buffer was added to the cells for thorough lysis. Luciferase activity (Promega) was detected. Neutralization efficiency was calculated by comparing the luciferase values of the experimental group with those of the virus-only control group. The calculation formula is as follows:

Percentage of inhibitory activity (%) = (virus control average value - test well reading value)/(virus control average value - cell control average value) x 100;

$IC_{50}$ values were calculated by Prism software.

2.4 Syncytium formation experiment

Hela cells were transfected with plasmids encoding the S proteins of SARS-CoV, MERS-CoV and SARS-CoV-2, respectively. At the same time, another group of Hela cells were transfected with plasmids encoding hACE2 and hDpp4, respectively. After 48h of transfection, Hela cells expressing S protein were mixed with different concentrations of antibodies and incubated at 37°C for 1 hour. The Hela cells expressing ACE2 were mixed with the above mixture of cells expressing S protein of SARS-CoV or SARS-CoV-2 and antibodies at a ratio of 1:1, and cultured at 37°C for 12 hours. Similarly, the Hela cells expressing hDpp4 were mixed with the above mixture of cells expressing MERS-CoV S protein and antibodies at a ratio of 1:1, and cultured at 37°C for 12 hours. 4% PFA was used to fix cells for 15min. Crystal violet staining was performed for 2 hours. Images were collected and analyzed using an Olympus IX73 confocal microscope.

2.5 Digestion inhibition experiment

Freshly purified S proteins of SARS-CoV, MERS-CoV and SARS-CoV-2were prepared, and incubated with 76E1 and control antibody 28-12 at room temperature according to 1:5 respectively for 1h; TPCK-Trypsin was added according to the mass ratio of Trypsin: HA = 1:100, and enzyme digestion was performed at room temperature (22°C). The time of adding Trypsin was recorded as 0 min, and the protein loading buffer was added immediately after taking out the mixture sample at 10min, 20min, and 40min respectively, then the sample was subjected to be boiled at 100°C for 10min. By Western Blot, 6xHis antibody was used to detect His development to judge HA protease digestion.

2.6 Euvirus neutralization assay

(1) Cell inoculation: Vero-E6 cells (novel coronavirus) or RD cells (OC43) in logarithmic growth phase were taken and inoculated into a 96-well plate, 100 μl per well and $4 \times 10^4$ cells per well.
(2) Neutralization experiment:
Dilution of test sample: 60 μl of 10 μg/ml pre-diluted sample was added in column 1 (B-G rows) of the 96-well plate. After addition of 60 μl of virus diluent, the final concentration of antibody was 5 μg/ml, and 60 μl of 3-fold diluted sample was added in turn to the remaining wells. The first line was cell control (CC) added with 120 μl of serum-free medium, and the eighth line was virus control (VC) added with 60 μl of serum-free medium.

Virus dilution: the titer of the virus storage solution was $2.5 \times 10^5$ TCID50/ml. 200 μl of virus storage solution was taken, and 25 ml of serum-free medium was added and mixed evenly, and the virus was diluted to 100 TCID50/50μl.
Drop-wise addition of viruses: viruses were dropped vertically (except cell control) into a 96-well plate, with a loading volume of 60 μl/well, and the final virus-antibody mixture was 120 μl.
Neutralization: the cell culture plate added with the sample was placed on a shaker for mixing evenly, and placed in a 37°C incubator for neutralization for 1 hour. After completion of neutralization, the supernatant of the culture plate inoculated with the cells was aspirated, and then 100 μl/well of the virus-serum mixture was pipetted thereto, and the mixture was cultured in a $CO_2$ incubator at 37 °C for 1 hour to perform the infection. After completion of the virus infection, the supernatant of the culture plate was aspirated, and the plaque formation experiment sample was added to a maintenance medium (DMEM medium containing 2% FBS) containing 1% methylcellulose, and placed in a $CO_2$ incubator at 37°C for 72-96 hours. The results were observed and recorded with an inverted microscope at day 4, and the supernatant was discarded, and the plaque formation experiment sample was fixed with formaldehyde, and the plaque was calculated and analyzed by crystal violet staining. For testing 100% inhibition rate, CPE was observed with an inverted microscope at day 2 and the results were recorded.

2.7 Animal Protection Experiment

76E1 prevention experiments on mice:

The 10-week-old hACE transgenic female mice were placed in the biosafety level 3 laboratory animal experimental room in advance and divided into 3 groups, 4-6 mice/group, and 3 groups of mice were inoculated with 50 mg/kg, 150 mg/kg 76E1 antibody and PBS as control on day 0, respectively. After 24h, the mice were anesthetized with 0.5% sodium pentobarbital, and the mice were nasally challenged with $3.7 \times 10^4$ virus particles of SARS-CoV-2. Mice were weighed for 3 days continuously and euthanized on day 3. Lung tissues were collected by dissection for viral RNA determination and histopathological examination.
Viral RNA was extracted from lung tissue with Trizol reagent (Invitrogen) and reverse transcribed into cDNA using a reverse transcription kit (TIANGEN BIOTECH CO., LTD). Real-time quantitative PCR was performed using the Super Real Pre Mix Plus SYBR Green kit and the above SARS-CoV-2 N gene-specific primers to determine the viral RNA content in lung tissue.
Mouse lungs were fixed in 4% paraformaldehyde solution. Tissue paraffin sections were stained with hematoxylin-eosin (H&E). The sections were observed by an Olympus microscope.

2.8 Experimental results

(1) 76E1 antibody can only bind to the S2 region of SARS-CoV-2

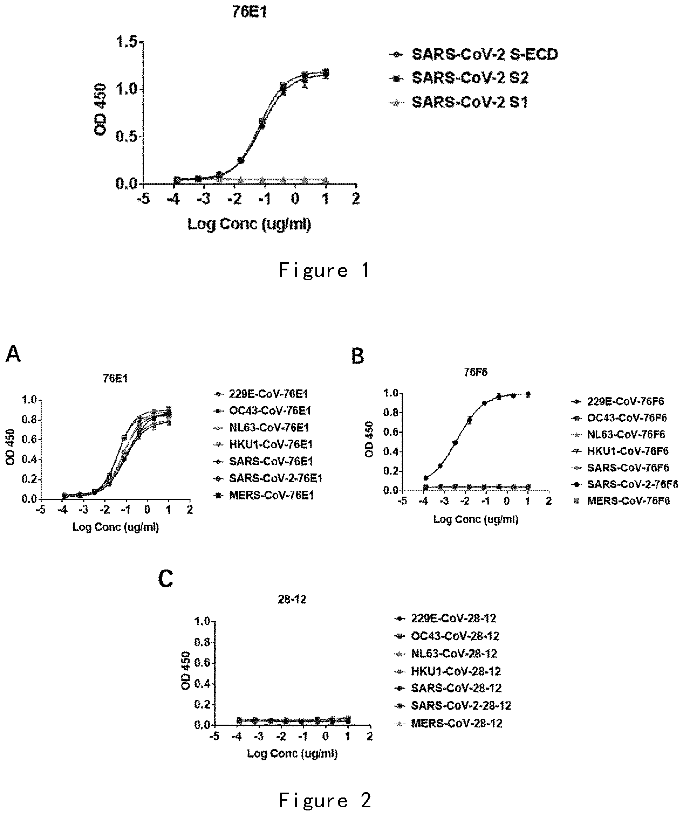

In order to study the binding of 76E1 antibody to SARS-CoV-2 S protein epitope position, the binding activity of 76E1 antibody to S full-length, S1 and S2 protein was verified by ELISA, respectively. As shown in Figure 1, the 76E1 antibody can bind to the S full-length protein as well as the S2 protein, but does not bind to S1. The 76E1 antibody, unlike most of the previously reported novel coronavirus antibodies targeting the receptor binding region, primarily targets S2, suggesting that its mechanism of action will also be different from that of antibodies targeting RBD.

(2) 76E1 antibody can bind to a broad spectrum of S proteins of different coronaviruses

The S2 region is located in the stem of the S protein, and its sequence is highly conserved among different coronaviruses. In order to study the ability of 76E1 antibody to bind to different coronavirus S proteins, the binding activity of 76E1 antibody to different soluble coronavirus S proteins purified *in vitro* was verified by ELISA. 76F6, a RBD-targeting antibody was used as a negative control antibody. A strain of anti-influenza fully human antibody 28-12 possessed by the inventors' laboratory served as a negative isotype control antibody. As shown in Figures 2A-C, the 76E1 antibody can broadly bind to the S proteins of 7 strains of coronaviruses that infect humans, including 229E-CoV, OC43-CoV, NL63-CoV, HKU1-CoV, SARS-CoV-2, SARS-CoV and MERS-CoV. RBD antibody 76F6 can only bind to SARS-CoV-2, while the negative control antibody 28-12 does not bind to various coronavirus S proteins.

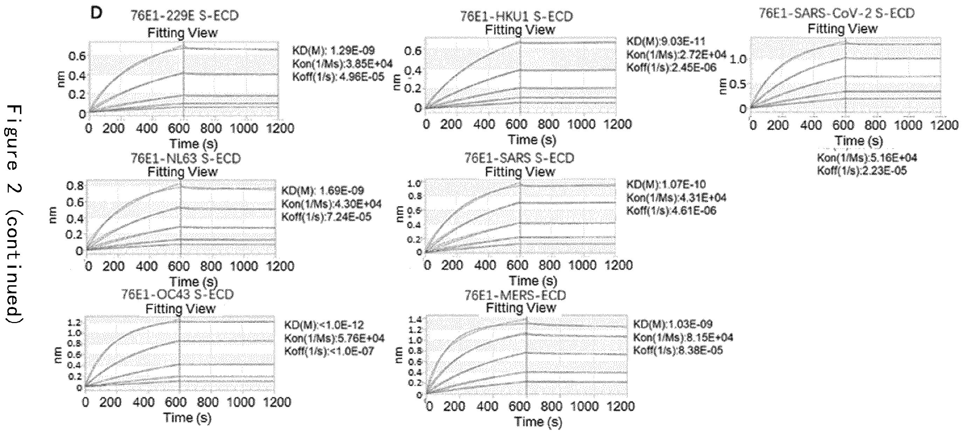

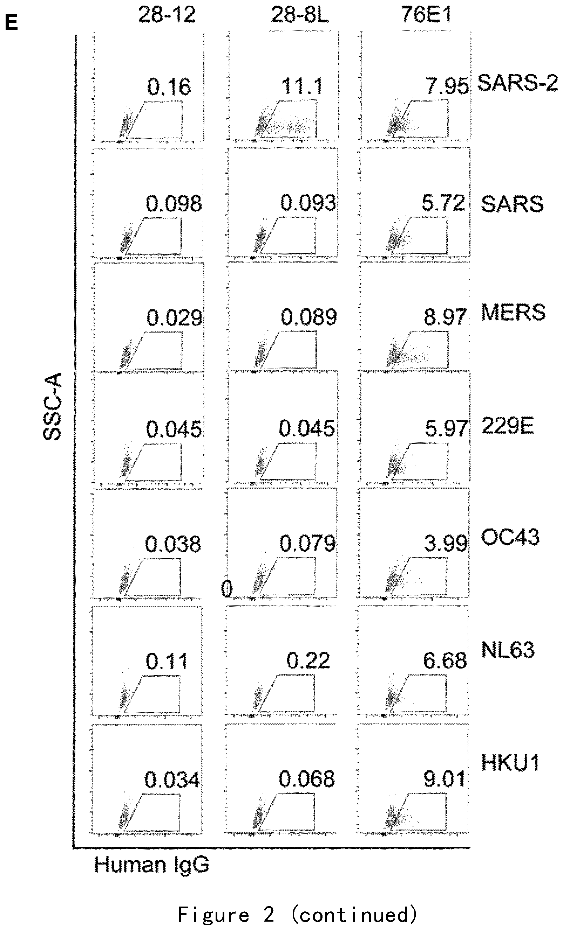

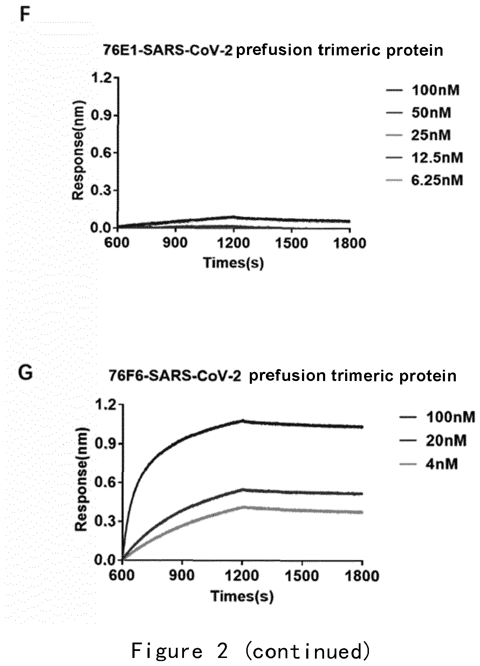

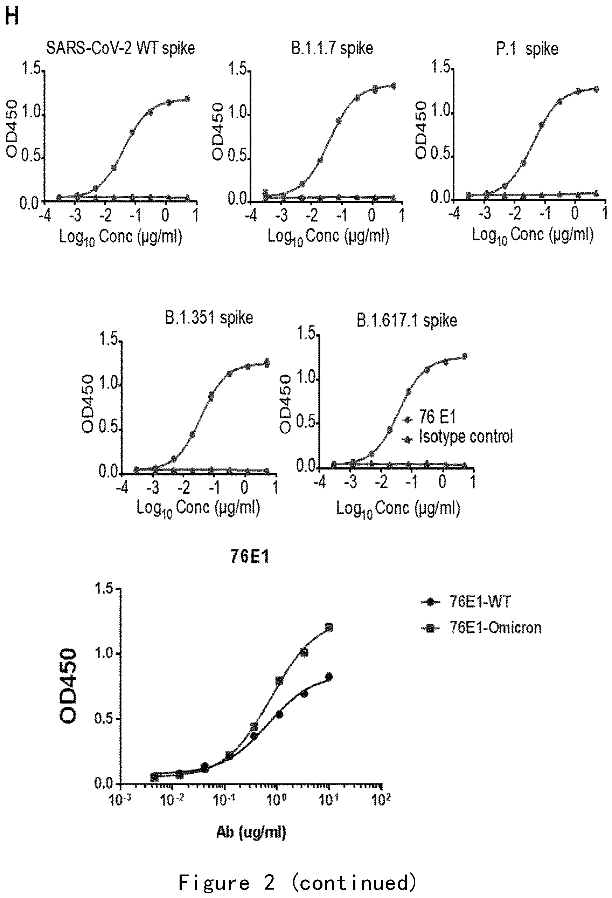

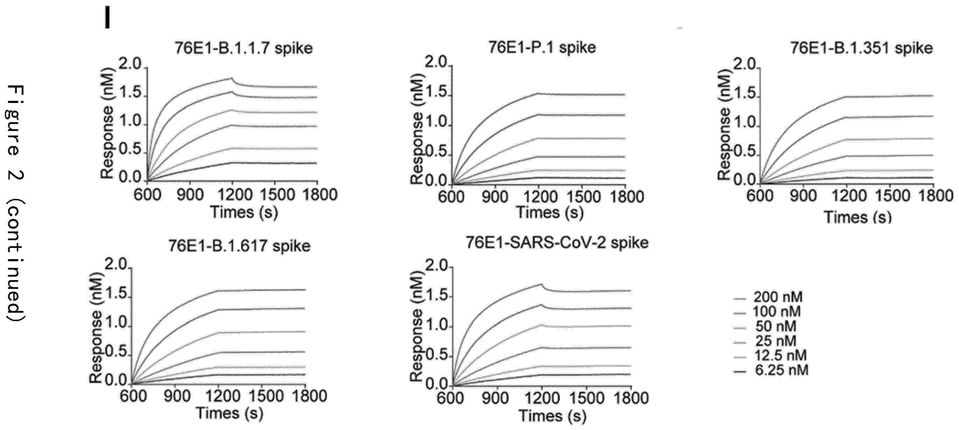

At the same time, the affinity of S proteins of the above 7 strains of coronaviruses purified *in vitro* with 76E1 was analyzed by bio-layer interference technique. 76E1 shows high affinity activity with S proteins of 7 strains of coronaviruses, reaching the level of nM or above (Figure 2, D). In order to further study the binding activity of 76E1 to S protein on the cell surface, CHO cells were transfected with different S proteins, and the binding activity of 76E1 to S protein on the cell membrane surface was detected by flow cytometry. The results show that 76E1 can bind to all seven S proteins expressed on cell surface, while 76F6 can only bind to SARS-CoV-2 S protein (Figure 2, E). However, 76E1 does not bind to the pre-fusion form of the trimeric S protein, implying that it recognizes one of the forms involved in the conformational change process of the S protein (Figure 2, F, G). As shown in Figure 2H, the 76E1 antibody can bind to the S protein of a variety of novel coronavirus mutant strains, which can resist the virus mutation. As shown in Figure 2I, the 76E1 antibody binds to the S proteins of various novel coronavirus mutant strains with high affinity.

(3) 76 E1 antibody can broadly neutralize different coronavirus pseudoviruses.

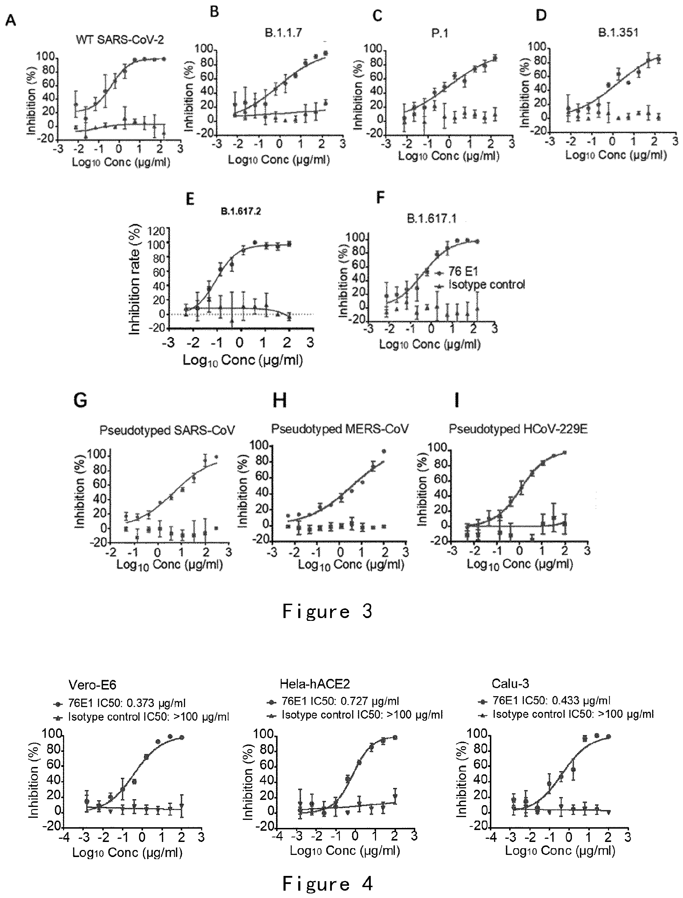

In order to further confirm the function of 76E1, pseudoviruses of SARS-CoV, MERS-CoV, SARS-CoV-2 and SARS-CoV-2 different mutant strains were packaged, then SARS-CoV and SARS-CoV-2 pseudovirus neutralization experiments were carried out on 293T cells which were stably transformed with human ACE2, and MERS-CoV and HCoV-229E pseudovirus neutralization experiments were carried out on huh7 cells. Experiments show that 76E1 neutralizes different strains of pseudoviruses with different abilities, and its neutralizing activity against SARS-CoV-2 and mutant strains thereof is the best, with the best $IC_{50}$ of 397 ng/ml (Figure 3, A-F), followed by the neutralizing activity against SARS-CoV, MARS- CoV and HCoV-229E (Figure 3, G-I).

(4) 76E1 antibody can neutralize SARS-CoV-2 envirus

To further confirm the neutralizing activity of 76E1 against SARS-CoV-2 envirus, SARS-CoV-2 envirus neutralizing activity assay was performed in BSL-3 laboratories. The results show that 76E1 can effectively inhibit infection of three susceptible cell lines by SARS-CoV-2 euvirus (Figure 4).

(5) 76E1 antibody recognizes the 809-833 and 809-823 linear epitopes of S2

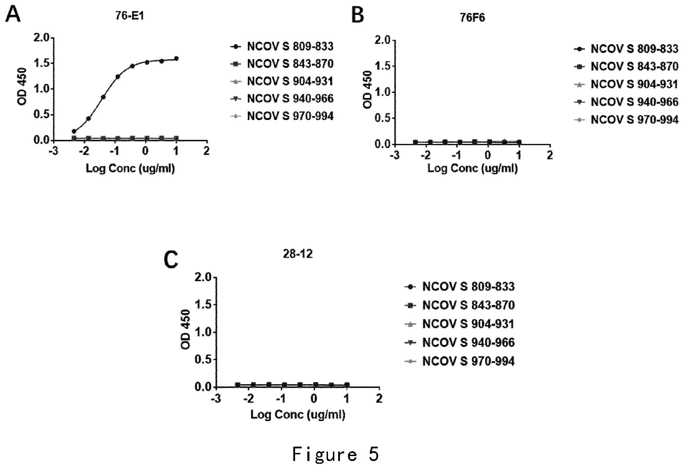

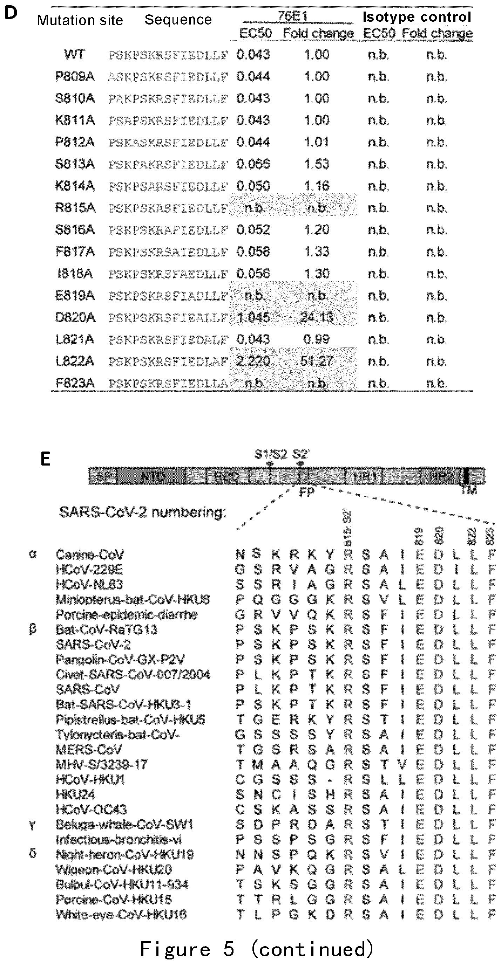

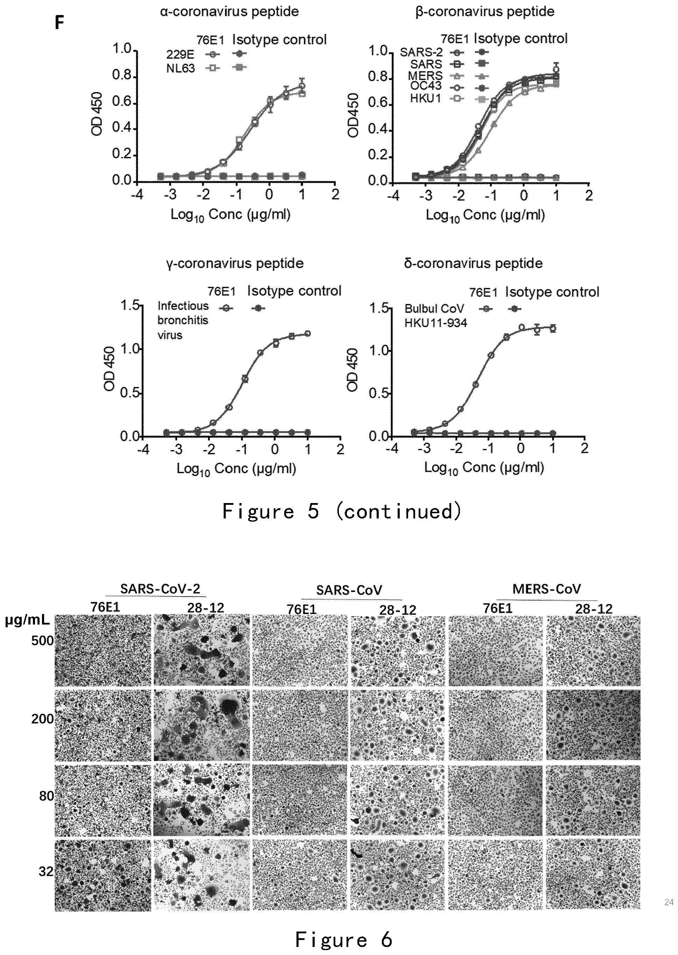

First, it was found by ELISA that 76E1 still maintained high binding activity to the denatured S protein, suggesting that 76E1 mainly recognized the linear epitope of S protein. Through the sequence alignment of S proteins of 7 strains of coronaviruses, it was found that the sequences of 6 positions on S2 were highly conserved. Since 76E1 can broadly bind to the S proteins of these 7 strains of viruses, the epitope of 76E1 is likely to be located on these 6 polypeptides. ELISA experiments show that 76E1 binds to the 809-833 polypeptide (Figure 5, A-C). By synthesizing the 809-823 amino acid polypeptide of truncated S protein and a series of polypeptides with each position individually mutated to alanine, it was found that the 815 (R), 819 (E) and 823 (F) were the most critical epitopes for the antibody 76E1 of the present invention, 820 (D) and 822 (L) were the secondary key epitopes, and 76E1 could bind to the 809-823 polypeptide of the present invention (Figure 5D).

The major epitope of 76E1 is therefore located on this polypeptide. This polypeptide is a fusion peptide, which plays an important role in mediating the fusion of viral envelope and host cell membrane. Moreover, this polypeptide is highly conserved in all coronaviruses (Figure 5E), and 76E1 can recognize the corresponding polypeptide sequences (corresponding to the 809-823 amino acids of the novel coronavirus Spike protein) of all four types (subfamily) of coronaviruses (Figure 5F).

(6) 76E1 antibody inhibits virus infection by inhibiting virus fusion with cell membrane

Since 76E1 binds to the S protein fusion peptide, it is hypothesized that 76E1 inhibits viral infection by inhibiting fusion of the virus to the cell membrane. By transfecting Hela cells with S plasmid and ACE2 plasmid respectively, the Hela-S cells and Hela-ACE2 cells were mixed and fused to simulate the fusion process of viral envelope and host cell membrane. The results show that for SARS-CoV-2 and SARS-CoV, 76E1 can effectively inhibit membrane fusion, while the control antibody 28-12 cannot inhibit membrane fusion (Figure 6). MERS-CoV mediated membrane fusion is less than SARS-CoV-2 and SARS-CoV, but 76E1 is still effective in inhibiting membrane fusion, whereas control antibody 28-12 is not (Figure 6). It is confirmed through the above experiments that 76E1 inhibits viral infection by inhibiting the fusion of virus and cell membrane.

(7) 76E1 inhibits membrane fusion by inhibiting enzyme cleavage at the enzyme cleavage site of S2'

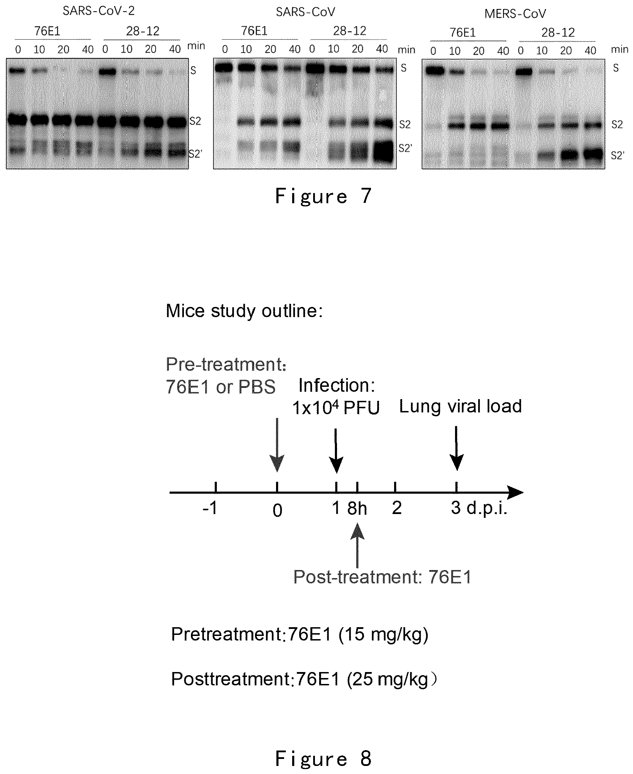

The S protein is cleaved by S1/S2 and S2' to expose the fusion peptide, thereby promoting the fusion of the virus with the cell membrane. Since the recognition epitope of 76E1 is close to the enzyme cleavage site of S2', it is speculated that 76E1 can inhibit the enzyme cleavage of S2'. Western blot experiments show that 76E1 can inhibit the enzyme cleavage of S protein by S2', while the control antibody can not (Figure 7).

(8) 76E1 antibody protects ACE2 humanized mice from SARS-CoV-2 envirus infection

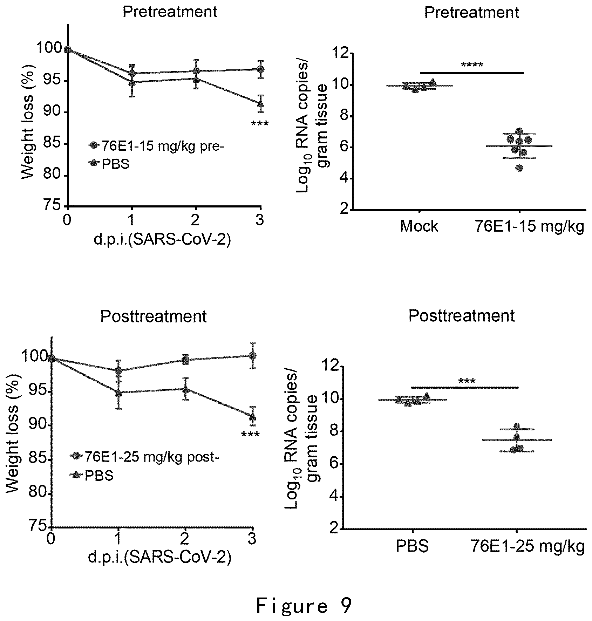

Therapeutic and prophylactic animal experiments were designed (Figure 8). The results show that 76E1 antibody reduces the weight loss of ACE2 humanized mice after infection with SARS-CoV-2 envirus, and 76E1 antibody reduces the viral load in the lungs of ACE2 humanized mice after infection with SARS-CoV-2 envirus (Figure 9).

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

**Claims**

1. A heavy chain variable region of an antibody comprising the following three complementarity determining regions (CDRs):

   CDR1 as shown in SEQ ID NO.: 3,
   CDR2 as shown in SEQ ID NO.: 4, and
   CDR3 as shown in SEQ ID NO.: 5;
   wherein, any one of the above amino acid sequences further includes a derivative sequence that is optionally added, deleted, modified and/or substituted with at least one amino acid and can retain the binding affinity of coronavirus S protein.

2. A heavy chain of an antibody, wherein the heavy chain has the heavy chain variable region of claim 1.

3. A light chain variable region of an antibody comprising the following three complementarity determining regions (CDRs):

   CDR1' as shown in SEQ ID NO.: 6,
   CDR2' with an amino acid sequence of EVN, and
   CDR3' as shown in SEQ ID NO.: 7;
   wherein, any one of the above amino acid sequences further includes a derivative sequence that is optionally added, deleted, modified and/or substituted with at least one amino acid and can retain the binding affinity of coronavirus S protein.

4. A light chain of an antibody, wherein the light chain has the light chain variable region of claim 3.

5. An antibody having:

(1) the heavy chain variable region of claim 1; and/or
(2) the light chain variable region of claim 3;
alternatively, the antibody has the heavy chain of claim 2; and/or the light chain of claim 4;
wherein, any one of the above amino acid sequences further includes a derivative sequence that is optionally added, deleted, modified and/or substituted with at least one amino acid and can retain the binding affinity of coronavirus S protein.

6. The antibody of claim 5, wherein the heavy chain variable region sequence of the antibody is as shown in SEQ ID NO.: 1; and the light chain variable region sequence of the antibody is as shown in SEQ ID NO.: 2;
in addition, the antibody has a heavy chain variable region sequence with an identity of ≥85% with the amino acid sequence shown in SEQ ID NO.: 1; and a light chain variable region sequence with an identity of ≥85% with the amino acid sequence shown in SEQ ID NO.: 2.

7. The antibody of claim 5, wherein the antibody is a specific anti-coronavirus antibody, preferably a specific anti-coronavirus S protein (preferably S2 protein) antibody.

8. A recombinant protein having:

(i) the heavy chain variable region of claim 1, the heavy chain of claim 2, the light chain variable region of claim 3, the light chain of claim 4, or the antibody of claim 5; and
(ii) optionally a tag sequence assisting expression and/or purification.

9. A CAR construct, wherein the scFv segment of the antigen-binding region of the CAR construct is a binding region that specifically binds to a coronavirus fusion protein, and the scFv has the heavy chain variable region of claim 1 and the light chain variable region of claim 3.

10. An antibody drug conjugate comprising:

(a) an antibody moiety selected from the group consisting of the heavy chain variable region of claim 1, the heavy chain of claim 2, the light chain variable region of claim 3, the light chain of claim 4, or the antibody of claim 5, and combinations thereof; and
(b) a coupling moiety conjugated to the antibody moiety, wherein the coupling moiety is selected from the group consisting of a detectable label, a drug, a toxin, a cytokine, a radionuclide, an enzyme, and combinations thereof.

11. Use of an active ingredient, wherein the active ingredient is selected from the group consisting of the heavy chain variable region of claim 1, the heavy chain of claim 2, the light chain variable region of claim 3, the light chain of claim 4, the antibody of claim 5, the recombinant protein of claim 8, and combinations thereof, wherein the active ingredient is used for the preparation of a medicament, a reagent, a test plate or a kit.

12. An epitope peptide, wherein the sequence of the epitope peptide comprises amino acids at positions 809-823 of SARS-CoV-2 S2 protein, preferably amino acids at positions 809-833 of SARS-CoV-2 S2 protein.

13. A vaccine composition comprising:

(i) a polypeptide comprising the amino acid sequence of positions 809-833 of the SARS-CoV-2 S2 protein as shown in SEQ ID NO.: 10 or a partial sequence thereof, wherein the partial sequence comprises the amino acid sequence of positions 809-823 of the SARS-CoV-2 S2 protein as shown in SEQ ID NO.: 11; and
(ii) a vaccine acceptable carrier.

14. An inhibitor targeting a linear epitope of SARS-CoV-2 S2 protein 809-823 or a linear epitope of SARS-CoV-2 S2 protein 809-833, for inhibiting novel coronavirus infection.

**15.** The inhibitor of claim 14, wherein the inhibitor is (a) a polypeptide comprising the amino acid sequence of positions 809-833 of the SARS-CoV-2 S2 protein as shown in SEQ ID NO.: 10 or a partial sequence thereof, wherein the partial sequence comprises the amino acid sequence of positions 809-823 of the SARS-CoV-2 S2 protein as shown in SEQ ID NO.: 11.

**16.** A method of treating coronavirus infection which comprises: administering the antibody of claim 5, the antibody-drug conjugate thereof, or the CAR-T cell expressing the antibody, and combinations thereof to a subject in need.

Figure 1

Figure 2

Figure 2 (continued)

Figure 2 (continued)

**F**

76E1-SARS-CoV-2 prefusion trimeric protein

**G**

76F6-SARS-CoV-2 prefusion trimeric protein

Figure 2 (continued)

**H**

Figure 2 (continued)

Figure 2 (continued)

Figure 3

Figure 4

Figure 5

**D**

| Mutation site | Sequence | 76E1 EC50 | 76E1 Fold change | Isotype control EC50 | Isotype control Fold change |
|---|---|---|---|---|---|
| WT | PSKPSKRSFIEDLLF | 0.043 | 1.00 | n.b. | n.b. |
| P809A | ASKPSKRSFIEDLLF | 0.044 | 1.00 | n.b. | n.b. |
| S810A | PAKPSKRSFIEDLLF | 0.043 | 1.00 | n.b. | n.b. |
| K811A | PSAPSKRSFIEDLLF | 0.043 | 1.00 | n.b. | n.b. |
| P812A | PSKASKRSFIEDLLF | 0.044 | 1.01 | n.b. | n.b. |
| S813A | PSKPAKRSFIEDLLF | 0.066 | 1.53 | n.b. | n.b. |
| K814A | PSKPSARSFIEDLLF | 0.050 | 1.16 | n.b. | n.b. |
| R815A | PSKPSKASFIEDLLF | n.b. | n.b. | n.b. | n.b. |
| S816A | PSKPSKRAFIEDLLF | 0.052 | 1.20 | n.b. | n.b. |
| F817A | PSKPSKRSAIEDLLF | 0.058 | 1.33 | n.b. | n.b. |
| I818A | PSKPSKRSFAEDLLF | 0.056 | 1.30 | n.b. | n.b. |
| E819A | PSKPSKRSFIADLLF | n.b. | n.b. | n.b. | n.b. |
| D820A | PSKPSKRSFIEALLF | 1.045 | 24.13 | n.b. | n.b. |
| L821A | PSKPSKRSFIEDALF | 0.043 | 0.99 | n.b. | n.b. |
| L822A | PSKPSKRSFIEDLAF | 2.220 | 51.27 | n.b. | n.b. |
| F823A | PSKPSKRSFIEDLLA | n.b. | n.b. | n.b. | n.b. |

**E**

SARS-CoV-2 numbering:

| | | | | | | | 815-S2' | | | | | 819 | 820 | | 822 | 823 |
|---|---|---|---|---|---|---|---|---|---|---|---|---|---|---|---|---|
| α | Canine-CoV | N S K R K Y R S A I E D L L F |
| | HCoV-229E | G S R V A G R S A I E D I L F |
| | HCoV-NL63 | S S R I A G R S A L E D L L F |
| | Miniopterus-bat-CoV-HKU8 | P Q G G G K R S V L E D L L F |
| | Porcine-epidemic-diarrhe | G R V V Q K R S F I E D L L F |
| β | Bat-CoV-RaTG13 | P S K P S K R S F I E D L L F |
| | SARS-CoV-2 | P S K P S K R S F I E D L L F |
| | Pangolin-CoV-GX-P2V | P S K P S K R S F I E D L L F |
| | Civet-SARS-CoV-007/2004 | P L K P T K R S F I E D L L F |
| | SARS-CoV | P L K P T K R S F I E D L L F |
| | Bat-SARS-CoV-HKU3-1 | P S K P T K R S F I E D L L F |
| | Pipistrellus-bat-CoV-HKU5 | T G E R K Y R S T I E D L L F |
| | Tylonycteris-bat-CoV- | G S S S S Y R S A I E D L L F |
| | MERS-CoV | T G S R S A R S A I E D L L F |
| | MHV-S/3239-17 | T M A A Q G R S T V E D L L F |
| | HCoV-HKU1 | C G S S S - R S L L E D L L F |
| | HKU24 | S N C I S H R S A I E D L L F |
| | HCoV-OC43 | C S K A S S R S A I E D L L F |
| γ | Beluga-whale-CoV-SW1 | S D P R D A R S T I E D L L F |
| | Infectious-bronchitis-vi | P S S P S G R S F I E D L L F |
| δ | Night-heron-CoV-HKU19 | N N S P Q K R S V I E D L L F |
| | Wigeon-CoV-HKU20 | P A V K Q G R S A L E D L L F |
| | Bulbul-CoV-HKU11-934 | T S K S G G R S A I E D L L F |
| | Porcine-CoV-HKU15 | T T R L G G R S A I E D L L F |
| | White-eye-CoV-HKU16 | T L P G K D R S A I E D L L F |

Figure 5 (continued)

Figure 5 (continued)

Figure 6

Figure 7

Mice study outline:

Pre-treatment:
76E1 or PBS    Infection:
               1x10⁴ PFU    Lung viral load

Post-treatment: 76E1

Pretreatment:76E1 (15 mg/kg)

Posttreatment:76E1 (25 mg/kg )

Figure 8

Figure 9

## INTERNATIONAL SEARCH REPORT

| | International application No. |
|---|---|
| | **PCT/CN2022/070309** |

**A. CLASSIFICATION OF SUBJECT MATTER**

C07K 16/10(2006.01)i; C07K 7/04(2006.01)i; C07K 14/165(2006.01)i; C12N 15/13(2006.01)i; C12N 15/85(2006.01)i; A61K 39/42(2006.01)i; A61P 31/14(2006.01)i

According to International Patent Classification (IPC) or to both national classification and IPC

**B. FIELDS SEARCHED**

Minimum documentation searched (classification system followed by classification symbols)

C07K, C12N, A61K, A61P

Documentation searched other than minimum documentation to the extent that such documents are included in the fields searched

Electronic data base consulted during the international search (name of data base and, where practicable, search terms used)

CNABS, DWPI, SIPOABS, CNKI, 万方, WANFANG, ISI Web of Science, BAIDU: 冠状病毒, S蛋白, S2蛋白, 单克隆抗体, 全人抗体, SARS-CoV-2, S protein, S2 protein, antibody, 76E1等; GenBank, EMBL, 中国专利生物序列检索系统, China Patents Biological Sequence Search System, STN, full-text database and searched sequences: SEQ ID NOs: 1-11.

**C. DOCUMENTS CONSIDERED TO BE RELEVANT**

| Category* | Citation of document, with indication, where appropriate, of the relevant passages | Relevant to claim No. |
|---|---|---|
| PX | WO 2021194940 A1 (CHILDREN'S NATIONAL MEDICAL CENTER) 30 September 2021 (2021-09-30) see claims 1-87, and SEQ ID NO: 272 | 12-15 |
| PX | WO 2021231441 A1 (UNIV VIRGINIA PATENT FOUNDATION et al.) 18 November 2021 (2021-11-18) see claims 1-39, and SEQ ID NO: 31 | 12-15 |
| A | CN 111303280 A (THE ACADEMY OF MILITARY MEDICAL SCIENCES (AMMS) OF THE PLA ACADEMY OF MILITARY SCIENCE) 19 June 2020 (2020-06-19) see entire document | 1-16 |
| A | CN 111620946 A (JIANGSU PROVINCIAL CENTER FOR DISEASE CONTROL AND PREVENTION; PUBLIC HEALTH RESEARCH INSTITUTE OF JIANGSU PROVINCE) 04 September 2020 (2020-09-04) see entire document | 1-16 |
| A | CN 111995677 A (WEIFANG MEDICAL UNIVERSITY) 27 November 2020 (2020-11-27) see entire document | 1-16 |

☑ Further documents are listed in the continuation of Box C. ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **29 January 2022** | **30 March 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/070309**

**C.    DOCUMENTS CONSIDERED TO BE RELEVANT**

| Category* | Citation of document, with indication, where appropriate, of the relevant passages | Relevant to claim No. |
|---|---|---|
| A | CN 111978378 A (WUHAN UNIVERSITY) 24 November 2020 (2020-11-24) see entire document | 1-16 |
| A | CN 111848789 A (CUSABIO BIOTECH CO., LTD.) 30 October 2020 (2020-10-30) see entire document | 1-16 |
| A | CN 111647053 A (THE ACADEMY OF MILITARY MEDICAL SCIENCES OF THE ACADEMY OF MILITARY SCIENCE, PHYSIOME INSTITUTE) 11 September 2020 (2020-09-11) see entire document | 1-16 |
| A | CN 111909260 A (CHONGQING MEDICAL UNIVERSITY, FENG, Yulin) 10 November 2020 (2020-11-10) see entire document | 1-16 |

Form PCT/ISA/210 (second sheet) (January 2015)

## INTERNATIONAL SEARCH REPORT

International application No.

**PCT/CN2022/070309**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

## INTERNATIONAL SEARCH REPORT

International application No.

**PCT/CN2022/070309**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **16**
because they relate to subject matter not required to be searched by this Authority, namely:

    [1]   PCT Rule 39.1(iv)–a method for treatment of a human or animal body by surgery or therapy.

    [2]   A search for claim 16 is formed on the basis of the following reasonably expected subject matter: a use of the antibody of claim 5, an antibody-drug conjugate of the antibody, or a CAR-T cell expressing the antibody, or a combination thereof in the preparation of drugs for treating coronavirus infection.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

INTERNATIONAL SEARCH REPORT
Information on patent family members

International application No.

**PCT/CN2022/070309**

| Patent document cited in search report | | | Publication date (day/month/year) | Patent family member(s) | | | Publication date (day/month/year) |
|---|---|---|---|---|---|---|---|
| WO | 2021194940 | A1 | 30 September 2021 | None | | | |
| WO | 2021231441 | A1 | 18 November 2021 | None | | | |
| CN | 111303280 | A | 19 June 2020 | None | | | |
| CN | 111620946 | A | 04 September 2020 | None | | | |
| CN | 111995677 | A | 27 November 2020 | CN | 111995676 | A | 27 November 2020 |
| | | | | CN | 111995678 | A | 27 November 2020 |
| | | | | CN | 112010966 | A | 01 December 2020 |
| | | | | CN | 111961133 | A | 20 November 2020 |
| | | | | CN | 112010965 | A | 01 December 2020 |
| | | | | CN | 111995675 | A | 27 November 2020 |
| CN | 111978378 | A | 24 November 2020 | None | | | |
| CN | 111848789 | A | 30 October 2020 | None | | | |
| CN | 111647053 | A | 11 September 2020 | None | | | |
| CN | 111909260 | A | 10 November 2020 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *J.biol.chem,* 1968, vol. 243, 3558
- **KABAT E.A et al.** Sequences of proteins of immunological interest. NIH Publication, 1991, 91-3242

- **SAMBROOK et al.** Molecular Cloning: A Laboratory Manual. Cold Spring Harbor Laboratory Press, 1989